# EUROPEAN PATENT SPECIFICATION

(11) **EP 3 835 295 B1**
(45) Date of publication and mention of the grant of the patent: **01.07.2026**
(21) Application number: 19214827.8
(22) Date of filing: 10.12.2019
(51) Int. Cl.: C07D 401/14, C07D 471/04, H10K 85/60

(54) **ACRIDINE COMPOUND AND ORGANIC SEMICONDUCTING LAYER, ORGANIC ELECTRONIC DEVICE AND DISPLAY DEVICE COMPRISING THE SAME**
ACRIDIN-VERBINDUNG UND ORGANISCHE HALBLEITENDE SCHICHT, ORGANISCHE ELEKTRONISCHE VORRICHTUNG UND ANZEIGEVORRICHTUNG DAMIT
COMPOSÉ ACRIDINE ET COUCHE SEMICONDUCTRICE ORGANIQUE, DISPOSITIF ÉLECTRONIQUE ORGANIQUE ET DISPOSITIF D'AFFICHAGE LE COMPRENANT

(43) Date of publication of application: 16.06.2021
(73) Proprietor: Novaled GmbH, 01099 Dresden (DE)
(72) Inventor: Senkovskyy, Volodymyr, 01099 Dresden (DE); Scholz, Johannes, 01099 Dresden (DE)
(74) Representative: Michalski Hüttermann & Partner mbB

(56) References cited:
- WO-A1-2015/083948
- WO-A1-2020/126972
- CN-A- 106 279 148
- CN-A- 109 232 561
- US-A1- 2015 034 915
- US-A1- 2018 114 920

## Description

### Technical Field

The present invention relates to compounds comprising a substituted or unsubstituted acridine group, an organic semiconductor layer comprising at least one compound with a substituted or unsubstituted acridine group and an organic electronic device comprising the same. The invention further relates to a display device comprising the organic electronic device.

### Background Art

It is always an aim to find new compounds that may be suitable used for organic semiconductor layer or organic electronic device comprising the same. Organic electronic devices, such as organic light-emitting diodes OLEDs, which are self-emitting devices, have a wide viewing angle, excellent contrast, quick response, high brightness, excellent operating voltage characteristics, and color reproduction. A typical OLED comprises an anode, a hole transport layer HTL, an emission layer EML, an electron transport layer ETL, and a cathode, which are sequentially stacked on a substrate. In this regard, the HTL, the EML, and the ETL are thin films formed from organic compounds.

When a voltage is applied to the anode and the cathode, holes injected from the anode move to the EML, via the HTL, and electrons injected from the cathode move to the EML, via the ETL. The holes and electrons recombine in the EML to generate excitons. When the excitons drop from an excited state to a ground state, light is emitted. The injection and flow of holes and electrons should be balanced, so that an OLED having the above-described structure has excellent efficiency and/or a long lifetime.

CN 109 232 561 A refers to a compound for preparing an organic electroluminescence device as well as a luminescence material and a device. The compound comprises a dibenzo[c,h]acridine group and a 1,10-phenanthroline derivative group.

US 2015/034915 A1 refers to an organic light-emitting device comprising a compound with a dibenzo[c,h]acridine group.

WO 2015/083948 A1 refers to an organic light-emitting device comprising a compound with a benzoacridine group.

Performance of an organic light emitting diode may be affected by characteristics of the semiconductor layer, and among them, may be affected by characteristics of an organic material of the semiconductor layer.

Particularly, development of a semiconductor layer being capable of increasing electron mobility and simultaneously increasing electrochemical stability is needed so that the organic electronic device, such as an organic light emitting diode, may be applied to a largesize flat panel display.

Further, development of a semiconductor layer being capable to have an extended life span at higher current density and thereby at higher brightness is needed. In particular the development of an organic semiconductor materials or semiconductor layer is needed with respect to lowering the operating voltage, which is important for reducing power consumption and increasing battery life, for example of a mobile display device.

There remains always a need to find new compounds that may improve performance of organic semiconductor materials, semiconductor layers, as well as organic electronic devices thereof, in particular to achieve high efficiency and long lifetime at lower operating voltages. Thereby the power consumption may be decreased and battery life improved, for example of mobile electronic devices.

### DISCLOSURE

The subject-matter for which protection is sought is claimed in the claims.

An aspect of the present invention provides a compound in accordance with claim 1 represented by the following Formula I:

Ar¹ - L - Ar² (I)

wherein,
Ar¹ is selected from F1 or F2: 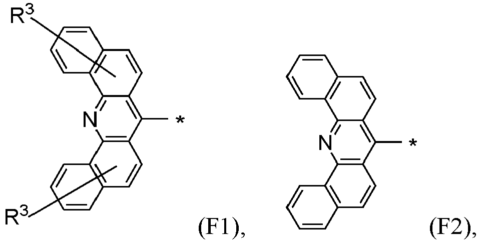 wherein
the asterisk symbol "*" represents the binding position;
R³ may be independently selected from H, C₆ to C₁₈ aryl, C₃ to C₂₀ heteroaryl, C₁ to C₁₆ alkyl, C₁ to C₁₆ alkoxy, C₃ to C₁₆ branched alkyl, C₃ to C₁₆ cyclic alkyl, C₃ to C₁₆ branched alkoxy, C₃ to C₁₆ cyclic alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy, -PX¹(R²)₂, D, F or CN;
   wherein R² is independently selected from C₆ to C₁₂ aryl, C₃ to C₁₂ heteroaryl, C₁ to C₁₆ alkyl, C₁ to C₁₆ alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy;
   X¹ is selected from S or O;

L is selected from substituted or unsubstituted phenylene, a substituted or unsubstituted biphenylene, a substituted or unsubstituted terphenylene, a substituted or unsubstituted anthracenylene, a substituted or unsubstituted naphthylene, a substituted or unsubstituted dibenzofuranylene, a substituted or unsubstituted dibenzothiophenylene, a substituted or unsubstituted carbazolylene, a substituted or unsubstituted pyridinylene, a substituted or unsubstituted phenylpyridinylene, a substituted or unsubstituted quinolinylene; and
   Ar² has the Formula IIa, IIc, IId or IIe, wherein
   the asterisk symbol "*" represents the binding position: 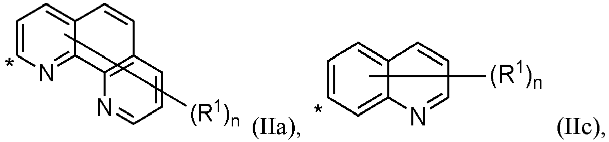 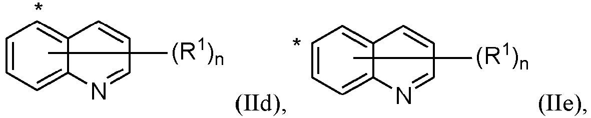 wherein
R¹ and substituents of Ar¹ and L are independently selected from H, C₆ to C₁₈ aryl, C₃ to C₂₀ heteroaryl, C₁ to C₁₆ alkyl, C₁ to C₁₆ alkoxy, C₃ to C₁₆ branched alkyl, C₃ to C₁₆ cyclic alkyl, C₃ to C₁₆ branched alkoxy, C₃ to C₁₆ cyclic alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy, -PX¹(R²)₂, D, F or CN; wherein
   R² is independently selected from C₆ to C₁₂ aryl, C₃ to C₁₂ heteroaryl, C₁ to C₁₆ alkyl, C₁ to C₁₆ alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy;
   X¹ is selected from S or O; and
      n is 0, 1, 2 or 3; and wherein Formula I comprises at least 9 to 25 aromatic rings.

Further embodiments are represented by the subclaims.

The compounds of Formula I offer lower operating voltage, which is important for reducing power consumption and increasing battery life. Moreover, the compounds of Formula I have a high Tg resulting in an improved thermal stability.

Hetero atoms, if not otherwise stated, may be individually selected from N, O, S, B, Si, P, Se, preferably from N, O and S, in addition preferred from S or O and more preferred is N.

If not otherwise stated the asterisk symbol "*" represents the binding position.

If not, otherwise stated H can represent hydrogen or deuterium.

If not otherwise stated the final syllable for group members of L is "ene".

According to another embodiment, wherein n is selected from 0, 1, 2 or 3, or preferably n may be selected 0, 1 or 2, or further preferred n may be selected 0 or 1, or in addition preferred n may be selected 1, also preferred n may be selected 0.

According to the present invention L is selected from substituted or unsubstituted phenylene, a substituted or unsubstituted biphenylene, a substituted or unsubstituted terphenylene, a substituted or unsubstituted anthracenylene, a substituted or unsubstituted naphthylene, a substituted or unsubstituted dibenzofuranylene, a substituted or unsubstituted dibenzothiophenylene, a substituted or unsubstituted carbazolylene, a substituted or unsubstituted pyridinylene, a substituted or unsubstituted phenylpyridinylene, a substituted or unsubstituted quinolinylene.

According to one embodiment L may be selected from an unsubstituted phenylene or an unsubstituted biphenylene.

According to one embodiment Ar¹ and Ar² are bonded in meta position to L.

According to one embodiment, wherein the compound is represented by the following Formula I:

Ar¹ - L - Ar² (I)

wherein,
Ar¹ may be selected from F1 or F2: 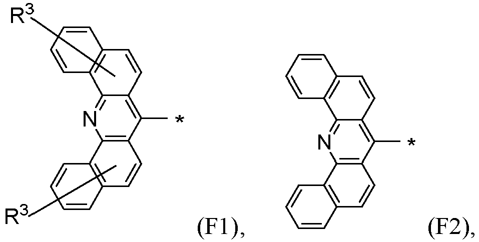 wherein
the asterisk symbol "*" represents the binding position;
R³ may be independently selected from H, C₆ to C₁₈ aryl, C₃ to C₂₀ heteroaryl, C₁ to C₁₆ alkyl, C₁ to C₁₆ alkoxy, C₃ to C₁₆ branched alkyl, C₃ to C₁₆ cyclic alkyl, C₃ to C₁₆ branched alkoxy, C₃ to C₁₆ cyclic alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy, -PX¹(R²)₂, D, F or CN;
   wherein R² is independently selected from C₆ to C₁₂ aryl, C₃ to C₁₂ heteroaryl, C₁ to C₁₆ alkyl, C₁ to C₁₆ alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy;
   X¹ is selected from S or O;
;
L may be selected from substituted or unsubstituted phenylene, a substituted or unsubstituted biphenylene, a substituted or unsubstituted terphenylene, a substituted or unsubstituted anthracenylene, a substituted or unsubstituted naphthylene, a substituted or unsubstituted dibenzofuranylene, a substituted or unsubstituted dibenzothiophenylene, a substituted or unsubstituted carbazolylene, a substituted or unsubstituted pyridinylene, a substituted or unsubstituted phenylpyridinylene, a substituted or unsubstituted quinolinylene; and
Ar² has the Formula IIa, IIc, IId or IIe: 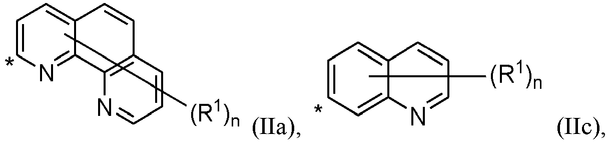 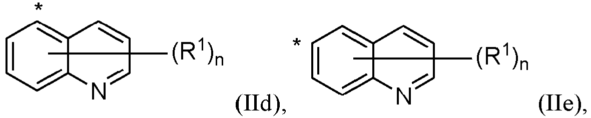 wherein
R¹ and substituents of Ar¹ and L are independently selected from H, C₆ to C₁₈ aryl, C₃ to C₂₀ heteroaryl, C₁ to C₁₆ alkyl, C₁ to C₁₆ alkoxy, C₃ to C₁₆ branched alkyl, C₃ to C₁₆ cyclic alkyl, C₃ to C₁₆ branched alkoxy, C₃ to C₁₆ cyclic alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy, -PX¹(R²)₂, D, F or CN; wherein
   R² is independently selected from C₆ to C₁₂ aryl, C₃ to C₁₂ heteroaryl, C₁ to C₁₆ alkyl, C₁ to C₁₆ alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy;
   X¹ may be selected from S or O; and
n is 0, 1, 2 or 3; wherein
Ar¹ and Ar² are bonded in meta position to L; and
wherein Formula I comprises at least 9 to 25 aromatic rings.

According to one embodiment, wherein the compound is represented by the following Formula I:

Ar¹ - L - Ar² (I)

wherein,
Ar¹ may be selected from F1 or F2: 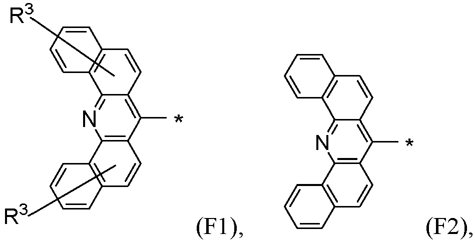 wherein
the asterisk symbol "*" represents the binding position;
R³ may be independently selected from H, C₆ to C₁₈ aryl, C₃ to C₂₀ heteroaryl, C₁ to C₁₆ alkyl, C₁ to C₁₆ alkoxy, C₃ to C₁₆ branched alkyl, C₃ to C₁₆ cyclic alkyl, C₃ to C₁₆ branched alkoxy, C₃ to C₁₆ cyclic alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy, -PX¹(R²)₂, D, F or CN;
   wherein R² is independently selected from C₆ to C₁₂ aryl, C₃ to C₁₂ heteroaryl, C₁ to C₁₆ alkyl, C₁ to C₁₆ alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy;
   X¹ is selected from S or O;
   ;
L may be selected from the group comprising a substituted or unsubstituted phenylene, a substituted or unsubstituted biphenylene, a substituted or unsubstituted terphenylene, a substituted or unsubstituted anthracenylene, a substituted or unsubstituted naphthylene, a substituted or unsubstituted dibenzofuranylene, a substituted or unsubstituted dibenzothiophenylene, a substituted or unsubstituted carbazolylene, a substituted or unsubstituted pyridinylene, a substituted or unsubstituted phenylpyridinylene, a substituted or unsubstituted quinolinylene; and
Ar² has the Formula IIa: 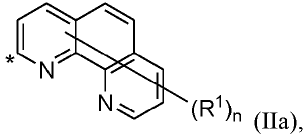 wherein
R¹ and substituents of Ar¹ and L are independently selected from H, C₆ to C₁₈ aryl, C₃ to C₂₀ heteroaryl, C₁ to C₁₆ alkyl, C₁ to C₁₆ alkoxy, C₃ to C₁₆ branched alkyl, C₃ to C₁₆ cyclic alkyl, C₃ to C₁₆ branched alkoxy, C₃ to C₁₆ cyclic alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy, -PX¹(R²)₂, D, F or CN; wherein
   R² is independently selected from C₆ to C₁₂ aryl, C₃ to C₁₂ heteroaryl, C₁ to C₁₆ alkyl, C₁ to C₁₆ alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy;
   X¹ may be selected from S or O; and
n is 0, 1, 2 or 3; wherein
Ar¹ and Ar² are bonded in meta position to L; and
wherein Formula I comprises at least 9 to 25 aromatic rings.

According to one embodiment, wherein the compound is represented by the following Formula I:

Ar¹ - L - Ar² (I)

wherein,
Ar¹ may be selected from F1 or F2: 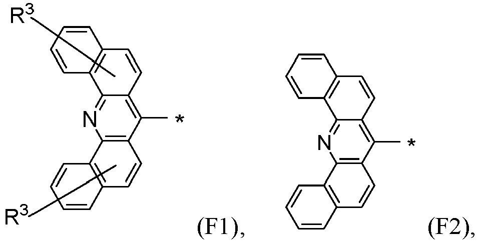 wherein
the asterisk symbol "*" represents the binding position;
R³ may be independently selected from H, C₆ to C₁₈ aryl, C₃ to C₂₀ heteroaryl, C₁ to C₁₆ alkyl, C₁ to C₁₆ alkoxy, C₃ to C₁₆ branched alkyl, C₃ to C₁₆ cyclic alkyl, C₃ to C₁₆ branched alkoxy, C₃ to C₁₆ cyclic alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy, -PX¹(R²)₂, D, F or CN;
   wherein R² is independently selected from C₆ to C₁₂ aryl, C₃ to C₁₂ heteroaryl, C₁ to C₁₆ alkyl, C₁ to C₁₆ alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy;
   X¹ is selected from S or O;
L may be selected from the group comprising a substituted or unsubstituted phenylene, a substituted or unsubstituted biphenylene, a substituted or unsubstituted terphenylene, a substituted or unsubstituted anthracenylene, a substituted or unsubstituted naphthylene, a substituted or unsubstituted dibenzofuranylene, a substituted or unsubstituted dibenzothiophenylene, a substituted or unsubstituted carbazolylene, a substituted or unsubstituted pyridinylene, a substituted or unsubstituted phenylpyridinylene, a substituted or unsubstituted quinolinylene; and
   Ar² has the Formula IIc, IId or IIe: 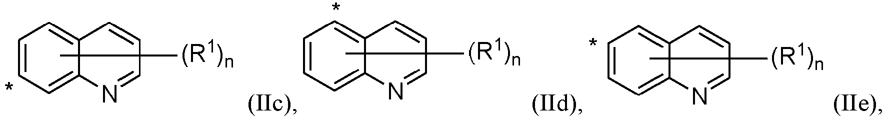
wherein
R¹ and substituents of Ar¹ and L are independently selected from H, C₆ to C₁₈ aryl, C₃ to C₂₀ heteroaryl, C₁ to C₁₆ alkyl, C₁ to C₁₆ alkoxy, C₃ to C₁₆ branched alkyl, C₃ to C₁₆ cyclic alkyl, C₃ to C₁₆ branched alkoxy, C₃ to C₁₆ cyclic alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy, -PX¹(R²)₂, D, F or CN; wherein
   R² is independently selected from C₆ to C₁₂ aryl, C₃ to C₁₂ heteroaryl, C₁ to C₁₆ alkyl, C₁ to C₁₆ alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy;
   X¹ may be selected from S or O; and
n is 0, 1, 2 or 3; and
wherein Formula I comprises at least 9 to 25 aromatic rings.

According to one embodiment, wherein the compound is represented by the following Formula I:

Ar¹ - L - Ar² (I)

wherein,
Ar¹ may be selected from F1 or F2: 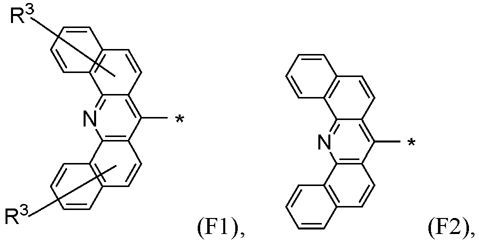 wherein
the asterisk symbol "*" represents the binding position;
R³ may be independently selected from H, C₆ to C₁₈ aryl, C₃ to C₂₀ heteroaryl, C₁ to C₁₆ alkyl, C₁ to C₁₆ alkoxy, C₃ to C₁₆ branched alkyl, C₃ to C₁₆ cyclic alkyl, C₃ to C₁₆ branched alkoxy, C₃ to C₁₆ cyclic alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy, -PX¹(R²)₂, D, F or CN;
   wherein R² is independently selected from C₆ to C₁₂ aryl, C₃ to C₁₂ heteroaryl, C₁ to C₁₆ alkyl, C₁ to C₁₆ alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy;
   X¹ is selected from S or O;;
L may be selected from the group comprising a substituted or unsubstituted phenylene, unsubstituted biphenylene, unsubstituted terphenylene, unsubstituted anthracenylene, unsubstituted dibenzofuranylene, unsubstituted dibenzothiophenylene, unsubstituted carbazolylene, unsubstituted pyridinylene, unsubstituted phenylpyridinylene, unsubstituted quinolinylene; and
Ar² may be selected from E1 to E4: 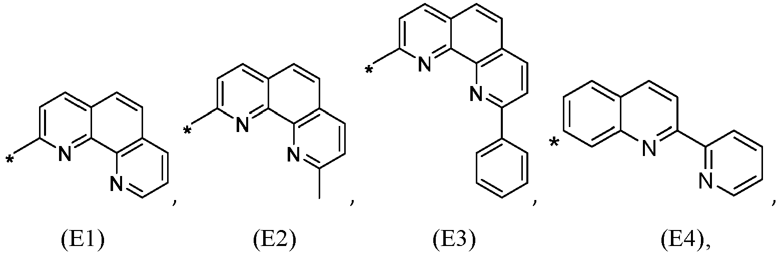
wherein, the asterisk symbol "*" represents the binding position; and wherein Ar¹ and Ar² are bonded in meta position to L; and wherein Formula I comprises at least 9 to 25 aromatic rings.

According to another embodiment, Ar¹ may be selected from F1 or F2: 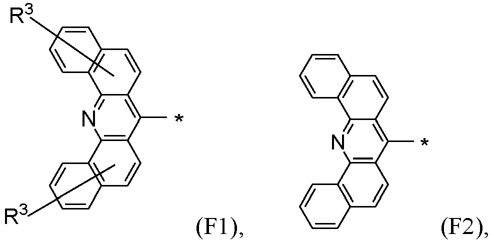 wherein the asterisk symbol "*" represents the binding position;
R³ may be independently selected from H, C₆ to C₁₈ aryl, C₃ to C₂₀ heteroaryl, C₁ to C₁₆ alkyl, C₁ to C₁₆ alkoxy, C₃ to C₁₆ branched alkyl, C₃ to C₁₆ cyclic alkyl, C₃ to C₁₆ branched alkoxy, C₃ to C₁₆ cyclic alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy, -PX¹(R²)₂, D, F or CN; wherein
X¹ may be selected from S or O; and
R² may be independently selected from C₆ to C₁₂ aryl, C₃ to C₁₂ heteroaryl, C₁ to C₁₆ alkyl, C₁ to C₁₆ alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy.

According to another embodiment, wherein the compound of Formula I may be represented by Formula Ia: 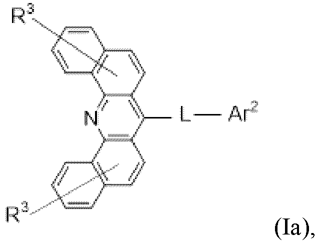 wherein
L may be selected from a substituted or unsubstituted phenylene, a substituted or unsubstituted biphenylene, a substituted or unsubstituted terphenylene, a substituted or unsubstituted anthracenylene, a substituted or unsubstituted naphthylene, a substituted or unsubstituted dibenzofuranylene, a substituted or unsubstituted dibenzothiophenylene, a substituted or unsubstituted carbazolylene, a substituted or unsubstituted pyridinylene, a substituted or unsubstituted phenylpyridinylene, a substituted or unsubstituted quinolinylene,
   Ar² may have the Formula IIa, IIc, IId or IIe: 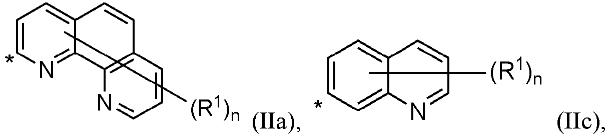 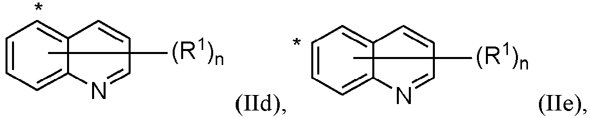 wherein
R¹, R³ and substituents L may be independently selected from H, C₆ to C₁₈ aryl, C₃ to C₂₀ heteroaryl, C₁ to C₁₆ alkyl, C₁ to C₁₆ alkoxy, C₃ to C₁₆ branched alkyl, C₃ to C₁₆ cyclic alkyl, C₃ to C₁₆ branched alkoxy, C₃ to C₁₆ cyclic alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy, -PX¹(R²)₂, D, F or CN; wherein
   R² is independently selected from C₆ to C₁₂ aryl, C₃ to C₁₂ heteroaryl, C₁ to C₁₆ alkyl, C₁ to C₁₆ alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy;
   X¹ is selected from S or O; and
n is 0, 1, 2 or 3; and
wherein Formula I comprises at least 9 to 25 aromatic rings.

According to another embodiment, wherein the compound of Formula I may be represented by Formula Ia: 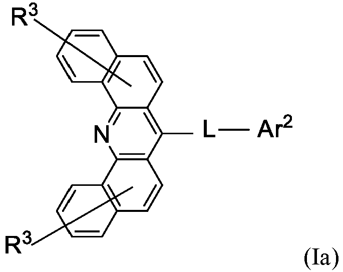 wherein,
L may be selected from a substituted or unsubstituted phenylene, a substituted or unsubstituted biphenylene, a substituted or unsubstituted terphenylene, a substituted or unsubstituted anthracenylene, a substituted or unsubstituted naphthylene, a substituted or unsubstituted dibenzofuranylene, a substituted or unsubstituted dibenzothiophenylene, a substituted or unsubstituted carbazolylene, a substituted or unsubstituted pyridinylene, a substituted or unsubstituted phenylpyridinylene, a substituted or unsubstituted quinolinylene,
   Ar² may have the Formula IIa, IIc, IId or IIe: 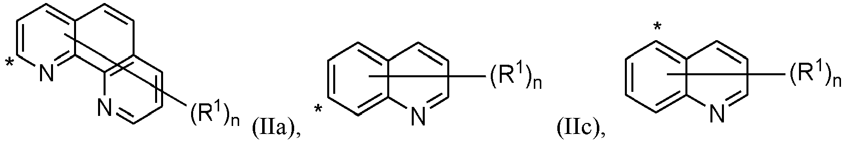 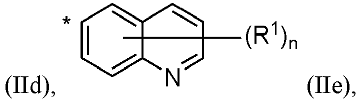 wherein
R¹, R³ and substituents L may be independently selected from H, C₆ to C₁₈ aryl, C₃ to C₂₀ heteroaryl, C₁ to C₁₆ alkyl, C₁ to C₁₆ alkoxy, C₃ to C₁₆ branched alkyl, C₃ to C₁₆ cyclic alkyl, C₃ to C₁₆ branched alkoxy, C₃ to C₁₆ cyclic alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy, -PX¹(R²)₂, D, F or CN; wherein
   R² is independently selected from C₆ to C₁₂ aryl, C₃ to C₁₂ heteroaryl, C₁ to C₁₆ alkyl, C₁ to C₁₆ alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy;
   X¹ may be selected from S or O; and
n is 0, 1, 2 or 3; and wherein Ar¹ and Ar² are bonded in meta position to L; and
   wherein Formula I comprises at least 9 to 25 aromatic rings. According to another embodiment, wherein the compound of Formula I may be represented by Formula Ib: 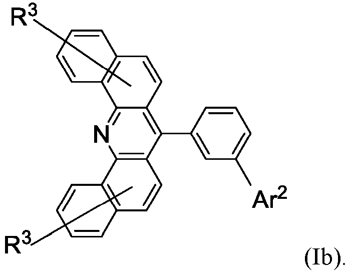 wherein
R³ may be independently selected from H, C₆ to C₁₈ aryl, C₃ to C₂₀ heteroaryl, C₁ to C₁₆ alkyl, C₁ to C₁₆ alkoxy, C₃ to C₁₆ branched alkyl, C₃ to C₁₆ cyclic alkyl, C₃ to C₁₆ branched alkoxy, C₃ to C₁₆ cyclic alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy, -PX¹(R²)₂, D, F or CN; wherein
   R² is independently selected from C₆ to C₁₂ aryl, C₃ to C₁₂ heteroaryl, C₁ to C₁₆ alkyl, C₁ to C₁₆ alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy; and
   X¹ may be selected from S or O; and n is 0, 1, 2 or 3; and wherein
Ar² may be selected from E1 to E4: 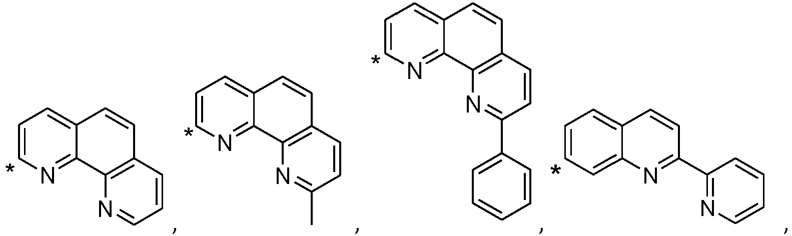 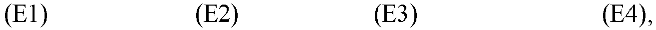 wherein the asterisk symbol "*" represents the binding position; and wherein Formula I comprises at least 9 to 25 aromatic rings.

According to another embodiment, wherein the compound of Formula I may be represented by Formula Ic: 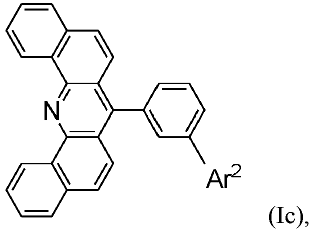 wherein
Ar² has the Formula IIa, IIc, IId or IIe, wherein the asterisk symbol "*" represents the binding position: 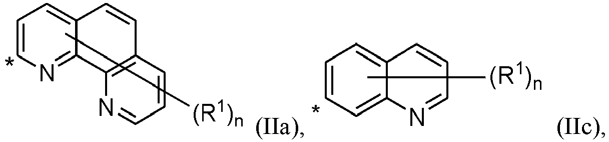 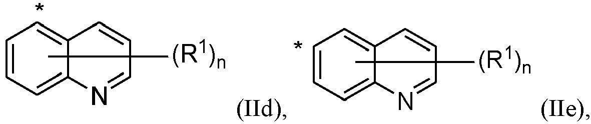 wherein R¹ may be independently selected from H, C₆ to C₁₈ aryl, C₃ to C₂₀ heteroaryl, C₁ to C₁₆ alkyl, C₁ to C₁₆ alkoxy, C₃ to C₁₆ branched alkyl, C₃ to C₁₆ cyclic alkyl, C₃ to C₁₆ branched alkoxy, C₃ to C₁₆ cyclic alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy, -PX¹(R²)₂, D, F or CN; wherein
   R² is independently selected from C₆ to C₁₂ aryl, C₃ to C₁₂ heteroaryl, C₁ to C₁₆ alkyl, C₁ to C₁₆ alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy; and
X¹ may be selected from S or O; and n is 0, 1, 2 or 3; and wherein Formula I comprises at least 9 to 25 aromatic rings.

According to another embodiment, wherein Ar² is selected from E1 to E4: 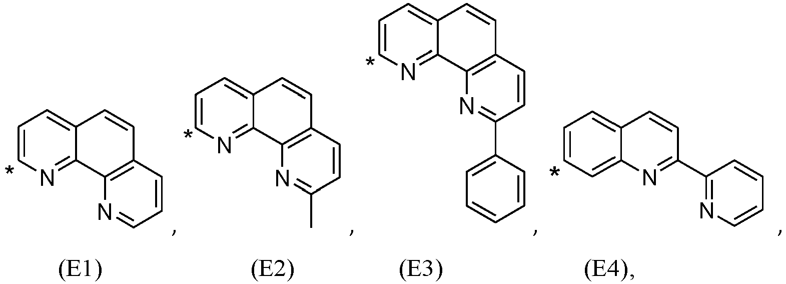 wherein the asterisk symbol "*" represents the binding position from Ar² to L.

According to another embodiment, wherein L is linkeded with Ar¹ and Ar² and L may be selected from the group comprising an unsubstituted phenylene, an unsubstituted biphenylene, an unsubstituted terphenylene, an unsubstituted anthracenylene, an unsubstituted naphthylene, an unsubstituted dibenzofuranylene, an unsubstituted dibenzothiophenylene, an unsubstituted carbazolylene, an unsubstituted pyridinylene, an unsubstituted phenylpyridinylene, an unsubstituted quinolinylene.

The term "L is linkeded" means that Ar¹ and Ar² are bonded to L in meta position.

According to another embodiment, wherein L is linkeded with Ar¹ and Ar² and L may be selected from the group comprising an unsubstituted phenylene, an unsubstituted biphenylene, an unsubstituted terphenylene, or an unsubstituted anthracenylene, an unsubstituted naphthylene.

According to another embodiment, wherein L is linkeded with Ar¹ and Ar² and L may be selected from the group comprising an unsubstituted dibenzofuranylene, an unsubstituted dibenzothiophenylene, an unsubstituted carbazolylene, an unsubstituted pyridinylene, an unsubstituted phenylpyridinylene, or an unsubstituted quinolinylene.

According to another embodiment, wherein L may be selected from B1 to B21: 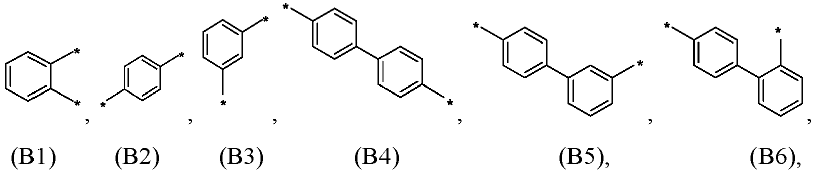 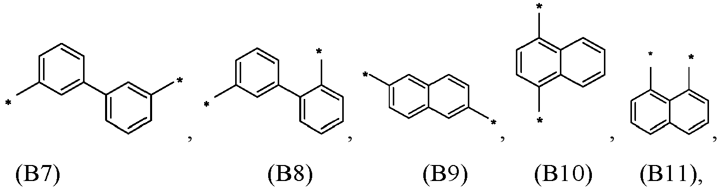 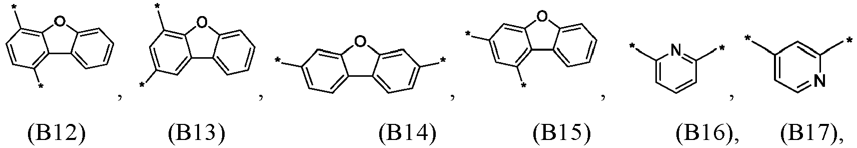 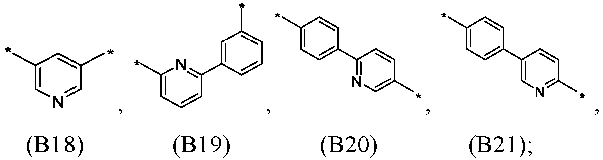 wherein the asterisk symbol "*" represents the binding position of L.

According to another embodiment, wherein L may be selected from B1 to B11. According to another embodiment, wherein L may be selected from B1 to B8. According to another embodiment, wherein L may be selected from B3, B5, B6, B7 or B8. According to another embodiment, wherein L may be selected from B1 to B3 and preferably from B3.

According to another embodiment, wherein R¹ may be selected from D1 to D8: 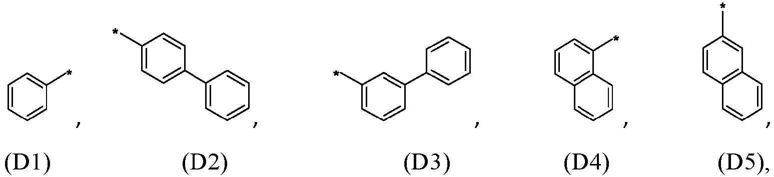 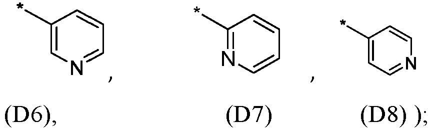 wherein the asterisk symbol "*" represents the binding position.

According to another embodiment, wherein the compound of Formula I may be selected from a compound of G1 to G20:

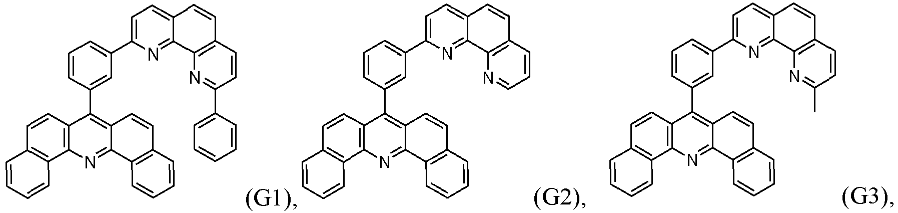

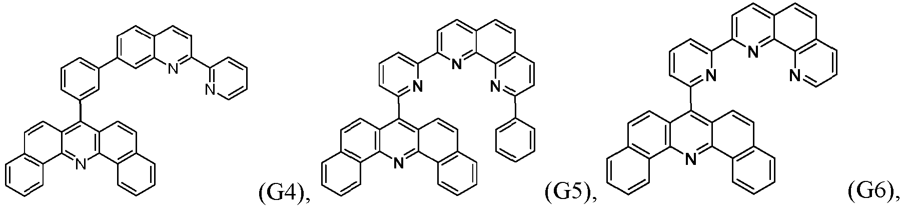

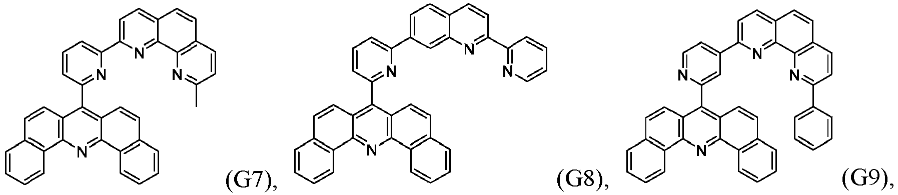

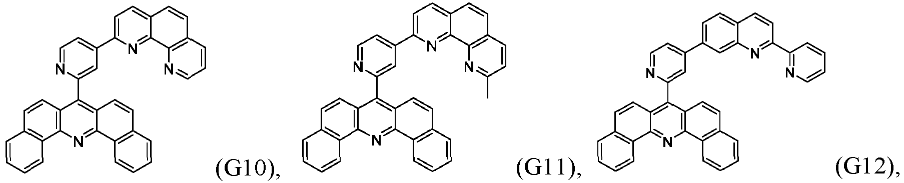

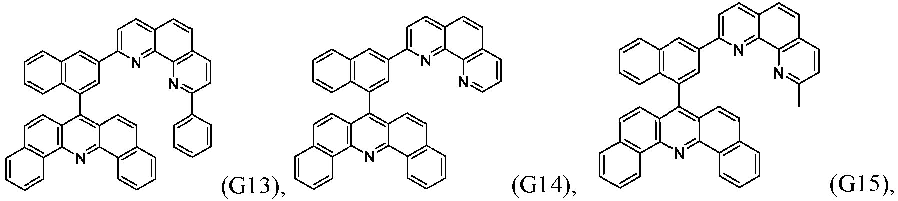

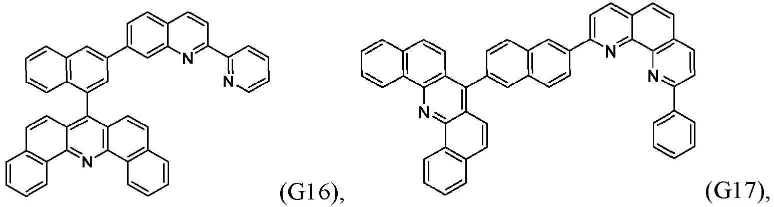

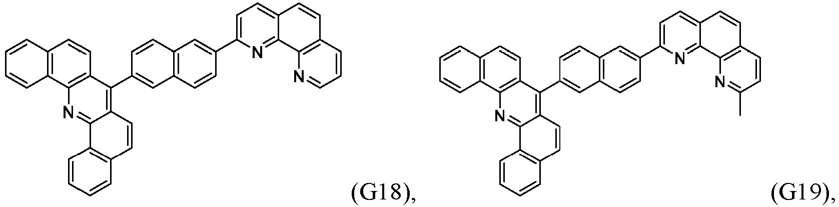

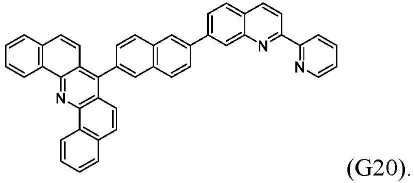

### Organic semiconductor layer

According to another aspect the present invention is directed to an organic electronic semiconductor layer that comprises at least one compound represented by Formula I.

According to another embodiment the organic electronic semiconductor layer comprises at least one compound represented by Formula I:

Ar¹ - L - Ar² (I)

wherein,
Ar¹ is selected from F1 or F2: 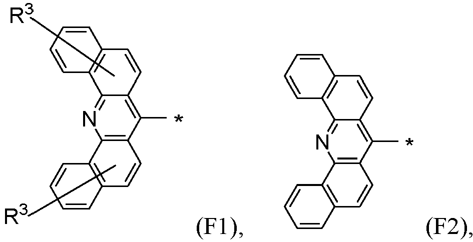 wherein
the asterisk symbol "*" represents the binding position;
R³ may be independently selected from H, C₆ to C₁₈ aryl, C₃ to C₂₀ heteroaryl, C₁ to C₁₆ alkyl, C₁ to C₁₆ alkoxy, C₃ to C₁₆ branched alkyl, C₃ to C₁₆ cyclic alkyl, C₃ to C₁₆ branched alkoxy, C₃ to C₁₆ cyclic alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy, -PX¹(R²)₂, D, F or CN;
   wherein R² is independently selected from C₆ to C₁₂ aryl, C₃ to C₁₂ heteroaryl, C₁ to C₁₆ alkyl, C₁ to C₁₆ alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy;
   X¹ is selected from S or O;
   ;

L is selected from substituted or unsubstituted phenylene, a substituted or unsubstituted biphenylene, a substituted or unsubstituted terphenylene, a substituted or unsubstituted anthracenylene, a substituted or unsubstituted naphthylene, a substituted or unsubstituted dibenzofuranylene, a substituted or unsubstituted dibenzothiophenylene, a substituted or unsubstituted carbazolylene, a substituted or unsubstituted pyridinylene, a substituted or unsubstituted phenylpyridinylene, a substituted or unsubstituted quinolinylene; and
Ar² has the Formula IIa, IIc, IId or IIe: 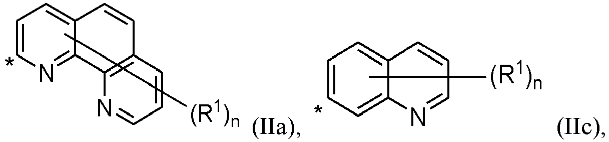 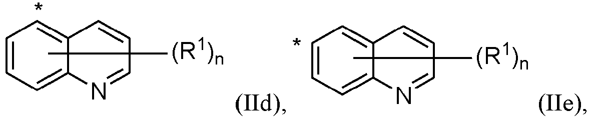 wherein
R¹ and substituents of Ar¹ and L are independently selected from H, C₆ to C₁₈ aryl, C₃ to C₂₀ heteroaryl, C₁ to C₁₆ alkyl, C₁ to C₁₆ alkoxy, C₃ to C₁₆ branched alkyl, C₃ to C₁₆ cyclic alkyl, C₃ to C₁₆ branched alkoxy, C₃ to C₁₆ cyclic alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy, -PX¹(R²)₂, D, F or CN; wherein
   R² is independently selected from C₆ to C₁₂ aryl, C₃ to C₁₂ heteroaryl, C₁ to C₁₆ alkyl, C₁ to C₁₆ alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy;
   X¹ is selected from S or O; and
n is 0, 1, 2 or 3; and wherein Formula I comprises at least 9 to 25 aromatic rings.

According to another aspect the present invention is directed to an organic electronic semiconductor layer that comprises at least one compound represented by Formula I.

According to another embodiment the organic electronic semiconductor layer comprises at least one compound represented by Formula I:

Ar¹- L - Ar² (I)

wherein,
Ar¹ is selected from F1 or F2: 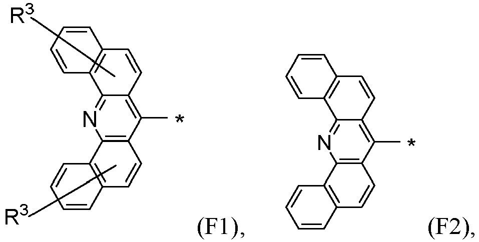 wherein
the asterisk symbol "*" represents the binding position;
L is selected from substituted or unsubstituted phenylene, a substituted or unsubstituted biphenylene, a substituted or unsubstituted terphenylene, a substituted or unsubstituted anthracenylene, a substituted or unsubstituted naphthylene, a substituted or unsubstituted dibenzofuranylene, a substituted or unsubstituted dibenzothiophenylene, a substituted or unsubstituted carbazolylene, a substituted or unsubstituted pyridinylene, a substituted or unsubstituted phenylpyridinylene, a substituted or unsubstituted quinolinylene; and
Ar² has the Formula IIa: 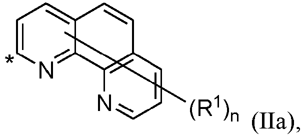 wherein
R¹ and substituents of Ar¹ and L are independently selected from H, C₆ to C₁₈ aryl, C₃ to C₂₀ heteroaryl, C₁ to C₁₆ alkyl, C₁ to C₁₆ alkoxy, C₃ to C₁₆ branched alkyl, C₃ to C₁₆ cyclic alkyl, C₃ to C₁₆ branched alkoxy, C₃ to C₁₆ cyclic alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy, -PX¹(R²)₂, D, F or CN; wherein
   R² is independently selected from C₆ to C₁₂ aryl, C₃ to C₁₂ heteroaryl, C₁ to C₁₆ alkyl, C₁ to C₁₆ alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy;
   X¹ is selected from S or O; and
n is 0, 1, 2 or 3; and wherein Formula I comprises at least 9 to 25 aromatic rings.

According to another aspect the present invention is directed to an organic electronic semiconductor layer that comprises at least one compound represented by Formula I.

According to another embodiment the organic electronic semiconductor layer comprises at least one compound represented by Formula I:

Ar¹ - L - Ar² (I)

wherein,
Ar¹ is selected from F1 or F2: 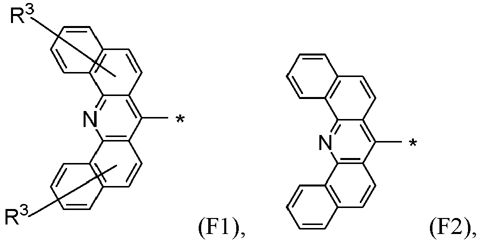 wherein
the asterisk symbol "*" represents the binding position;
R³ may be independently selected from H, C₆ to C₁₈ aryl, C₃ to C₂₀ heteroaryl, C₁ to C₁₆ alkyl, C₁ to C₁₆ alkoxy, C₃ to C₁₆ branched alkyl, C₃ to C₁₆ cyclic alkyl, C₃ to C₁₆ branched alkoxy, C₃ to C₁₆ cyclic alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy, -PX¹(R²)₂, D, F or CN;
   wherein R² is independently selected from C₆ to C₁₂ aryl, C₃ to C₁₂ heteroaryl, C₁ to C₁₆ alkyl, C₁ to C₁₆ alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy;
   X¹ is selected from S or O;
L is selected from substituted or unsubstituted phenylene, a substituted or unsubstituted biphenylene, a substituted or unsubstituted terphenylene, a substituted or unsubstituted anthracenylene, a substituted or unsubstituted naphthylene, a substituted or unsubstituted dibenzofuranylene, a substituted or unsubstituted dibenzothiophenylene, a substituted or unsubstituted carbazolylene, a substituted or unsubstituted pyridinylene, a substituted or unsubstituted phenylpyridinylene, a substituted or unsubstituted quinolinylene; and
   Ar² has the Formula, IIc; IId or IIe: 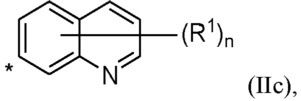 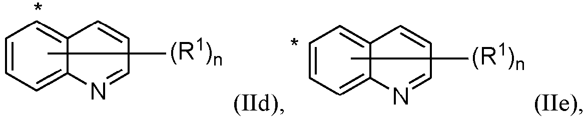 wherein
R¹ and substituents of Ar¹ and L are independently selected from H, C₆ to C₁₈ aryl, C₃ to C₂₀ heteroaryl, C₁ to C₁₆ alkyl, C₁ to C₁₆ alkoxy, C₃ to C₁₆ branched alkyl, C₃ to C₁₆ cyclic alkyl, C₃ to C₁₆ branched alkoxy, C₃ to C₁₆ cyclic alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy, -PX¹(R²)₂, D, F or CN; wherein
   R² is independently selected from C₆ to C₁₂ aryl, C₃ to C₁₂ heteroaryl, C₁ to C₁₆ alkyl, C₁ to C₁₆ alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy;
   X¹ is selected from S or O; and
n is 0, 1, 2 or 3; and wherein Formula I comprises at least 9 to 25 aromatic rings.

According to another embodiment the organic electronic semiconductor layer comprises at least one compound represented by Formula I, and a dopant, wherein the dopant is selected from the group comprising a metal, metal salt or organic metal complex.

According to another embodiment, the organic semiconductor layer and/or the organic semiconductor layer of the organic electronic device, wherein the dopant may be:
- a metal, preferably the metal is selected from Li, Na, Cs, Mg, Ca, Sr, Sm, Yb, and more preferred the metal is selected from Li, Cs, Mg, Yb, ;
- a metal salt, preferably the metal salt is selected from the group consisting of an alkali metal salts, alkaline earth metal salts, rare earth metal salts and mixtures thereof,
   wherein the alkali metal salt is preferably selected from the group consisting of LiF, LiCl, LiBr, LiI and mixtures thereof, and more preferred is LiF;
- a metal complex, preferably the metal complex is an organic alkali metal complex, preferably LiQ or alkali borate.

According to another embodiment, the metal may have an electronegativity of ≥ 0.7 to ≤ 1.3 according to Pauling scale, and preferably the metal dopant has an electronegativity of ≥ 0.9 to ≤ 1.2, further preferred ≥ 1 to ≤ 1.1.

According to another embodiment, the metal may be selected from the group comprising:
- alkali metals, alkaline earth metals and rare earth metals;
- Li, Na, Cs, Mg, Ca, Sr, Sm or Yb;
- Li, Cs, Mg or Yb; or
- Li or Yb.

According to another embodiment, the metal may be selected from Li, Na, Cs, Mg, Ca, Sr, Sm or Yb and more preferably the metal may be selected from Li, Cs, Mg or Yb.

According to another embodiment, the first organic semiconductor layer and/or the first organic semiconductor layer of the organic electronic device may comprise a metal dopant, wherein the metal dopant is a metal selected from Li or Yb.

According to one embodiment, wherein the organic electronic semiconductor layer may consists of a compound of Formula I and a metal, wherein the metal also named herein "metal dopant" is a metal from the group consisting of alkali metals, alkaline earth metals and rare earth metals preferably the metal may be selected from Li, Na, Cs, Mg, Ca, Sr, Sm or Yb and more preferably the metal may be selected from Li, Cs, Mg or Yb.

According to one embodiment, wherein the organic electronic semiconductor layer may consist of a compound of Formula I and a metal dopant, wherein the metal dopant is a metal selected from Li or Yb.

According to another embodiment, the dopant may be selected from alkali metal salts also known as alkali metal halides, are the family of inorganic compounds with the chemical formula MX, where M is an alkali metal and X is a halogen.
M can be selected from Li, Na, Potassium, Rubidium and Cesium.

X can be selected from F, Cl, Br and J.

According to various embodiments of the present invention a lithium halide may be preferred. The lithium halide can be selected from the group comprising LiF, LiCl, LiBr and LiJ. However, most preferred is LiF.

The alkali halide is essentially non-emissive or non-emissive.

According to another embodiment, the dopant may be selected from alkali organic complex, wherein the alkali organic complex comprises an alkali metal and at least one organic ligand. The alkali metal is preferably selected from lithium.
According to various embodiments of the present invention the organic ligand of the lithium organic complex is a quinolate, a borate, a phenolate, a pyridinolate or a Schiff base ligand;
- preferably the lithium quinolate complex has the formula III, IV or V: 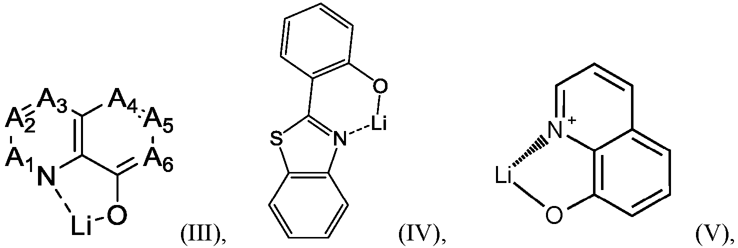 wherein
   A₁ to A₆ are same or independently selected from CH, CR, N and O;
   R is same or independently selected from hydrogen, halogen, alkyl or arylene or heteroarylene with 1 to 20 carbon atoms; and more preferred A1 to A6 are CH;
- preferably the borate based organic ligand is a tetra(1H-pyrazol-1-yl)borate;
- preferably the phenolate is a 2-(pyridin-2-yl)phenolate, a 2-(diphenylphosphoryl)phenolate, an imidazol phenolates, or 2-(pyridin-2-yl)phenolate and more preferred 2-(1-phenyl-1H-benzo[d]imidazol-2-yl)phenolate;
- preferably the pyridinolate is a 2-(diphenylphosphoryl)pyridin-3-olate.

According to various embodiments of the present invention the organic ligand of the alkali organic complex, preferably of a lithium organic complex, can be a quinolate. Quinolates that can be suitable used are disclosed in WO 2013079217 A1.

According to various embodiments of the present invention the organic ligand of the lithium organic complex can be a borate based organic ligand, Preferably the lithium organic complex is a lithium tetra(1H-pyrazol-1-yl)borate. Borate based organic ligands that can be suitable used are disclosed in WO 2013079676 A1.

According to various embodiments of the present invention the organic ligand of the lithium organic complex can be a phenolate ligand, Preferably the lithium organic complex is a lithium 2-(diphenylphosphoryl)phenolate. Phenolate ligands that can be suitable used are disclosed in WO 2013079678 A1.

Further, phenolate ligands can be selected from the group of pyridinolate, preferably 2-(diphenylphosphoryl)pyridin-3-olate. Pyridine phenolate ligands that can be suitable used are disclosed in JP 2008195623.

In addition, phenolate ligands can be selected from the group of imidazol phenolates, preferably 2-(1-phenyl-1H-benzo[d]imidazol-2-yl)phenolate. Imidazol phenolate ligands that can be suitable used are disclosed in JP 2001291593.

Also, phenolate ligands can be selected from the group of oxazol phenolates, preferably 2-(benzo[d]oxazol-2-yl)phenolate. Oxazol phenolate ligands that can be suitable used are disclosed in US 20030165711.

The alkali organic complex may be essentially non-emissive.

The compound of formula I and/or the metal, also named "metal dopant" herein may be essentially non-emissive.

The organic semiconductor layer comprising the composition of the present invention may be essentially non-emissive.

The thickness of the organic semiconductor layer may be from 0.5 nm to 100 nm, for example 2 nm to 40 nm. When the thickness of the organic semiconductor layer is within these ranges, the organic semiconductor layer may have improved charge transport ability without a substantial increase in operating voltage.

The organic semiconductor layer comprising the composition of the present invention may have strong electron transport characteristics to increase charge mobility and/or stability.

According to one embodiment of the present invention the organic semiconductor layer is an electron transport layer, wherein an electron transport layer may consists of a compound of Formula I.

According to one embodiment of the present invention the organic semiconductor layer is an electron transport layer, wherein an electron transport layer may consists of a compound of Formula I and a dopant.

According to one embodiment of the present invention the organic semiconductor layer is an electron transport layer and an optional second organic semiconductor layer is a hole injection layer.

According to one embodiment of the present invention the organic semiconductor layer is an electron transport layer and an optional second organic semiconductor layer is a n-type charge generation layer.

According to one embodiment of the present invention the organic semiconductor layer is an n-type charge generation layer, wherein an n-type charge generation layer may consists of a compound of Formula I.

According to one embodiment of the present invention the organic semiconductor layer is an n-type charge generation layer, wherein an n-type charge generation layer may consists of a compound of Formula I and a dopant.

According to one embodiment of the present invention the organic semiconductor layer is an n-type charge generation layer and an optional second organic semiconductor layer is a hole injection layer and/or a p-type charge generation layer.

According to one embodiment of the present invention the organic semiconductor layer is electron transport layer and/or n-type charge generation layer and an optional second organic semiconductor layer is a hole injection layer and/or a p-type charge generation layer.

### Electronic device

Another aspect of the present invention is directed to an organic electronic device.

According to one embodiment, the organic electronic device comprising an anode layer, a cathode layer and at least one organic semiconductor layer, wherein the at least one organic semiconductor layer comprises a compound of Formula I according to the present invention.

According to one embodiment, the organic electronic device comprising an anode, at least one organic semiconductor layer and a cathode; wherein at least one organic semiconductor layer comprises a compound represented by the following Formula I:

Ar¹ - L - Ar² (I)

wherein,
Ar¹ is selected from F1 or F2: 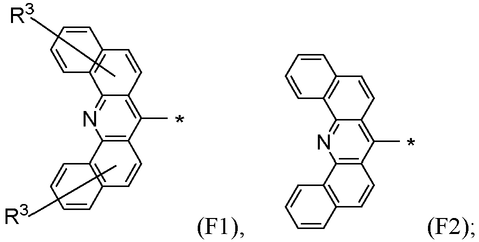 ;
L is selected from substituted or unsubstituted phenylene, a substituted or unsubstituted biphenylene, a substituted or unsubstituted terphenylene, a substituted or unsubstituted anthracenylene, a substituted or unsubstituted naphthylene, a substituted or unsubstituted dibenzofuranylene, a substituted or unsubstituted dibenzothiophenylene, a substituted or unsubstituted carbazolylene, a substituted or unsubstituted pyridinylene, a substituted or unsubstituted phenylpyridinylene, a substituted or unsubstituted quinolinylene; and
   Ar² has the Formula IIa, IIc, IId or IIe: 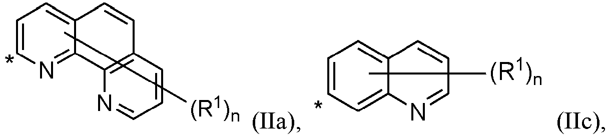 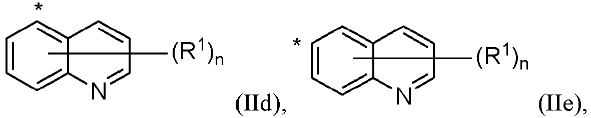 wherein
R¹ and substituents of Ar¹ and L are independently selected from H, C₆ to C₁₈ aryl, C₃ to C₂₀ heteroaryl, C₁ to C₁₆ alkyl, C₁ to C₁₆ alkoxy, C₃ to C₁₆ branched alkyl, C₃ to C₁₆ cyclic alkyl, C₃ to C₁₆ branched alkoxy, C₃ to C₁₆ cyclic alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy, -PX¹(R²)₂, D, F or CN; wherein
   R² is independently selected from C₆ to C₁₂ aryl, C₃ to C₁₂ heteroaryl, C₁ to C₁₆ alkyl, C₁ to C₁₆ alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy;
   X¹ is selected from S or O; and
   n is 0, 1, 2 or 3;and wherein Formula I comprises at least 9 to 25 aromatic rings, wherein the asterisk symbol "*" represents the binding position.

According to one embodiment, the organic electronic device comprising an anode, an organic semiconductor layer, a second organic semiconductor layer and a cathode; wherein the organic semiconductor layer may comprise a compound represented by Formula I; and a dopant, wherein the dopant is selected from the group comprising a metal, metal salt or organic metal complex.

### Method of manufacturing an organic electronic device

According to another aspect of the present invention, there is provided a method of manufacturing an organic electronic device, the method using:
- at least one deposition source, preferably two deposition sources and more preferred at least three deposition sources.

The methods for deposition that can be suitable comprise:
- deposition via vacuum thermal evaporation;
- deposition via solution processing, preferably the processing may be selected from spincoating, printing, casting; and/or
- slot-die coating.

According to various embodiments of the present invention, there is provided a method using:
- a deposition source to release the compound of Formula I according to the invention, and
- optional a second deposition source to release the at least one metal dopant;
- optional a third deposition source to release for example a radialene compound,
the method comprising the steps of forming the organic semiconductor layer; whereby for an organic light-emitting diode (OLED):
- the organic semiconductor layer is formed by releasing the compound of Formula I from the deposition source and the optional metal dopant from the second deposition source.

According to various embodiments of the present invention, the method may further include forming on the anode electrode an emission layer and at least one layer selected from the group consisting of forming a hole transport layer, or forming a hole blocking layer, between the anode electrode and the organic semiconductor layer.

According to various embodiments of the present invention, the method may further include the steps for forming an organic light-emitting diode (OLED), wherein
- on a substrate a anode electrode is formed,
- on the anode electrode a hole injection layer is formed, wherein a hole injection layer may comprise a radialene compound,
- on hole injection layer an emission layer is formed,
- on the emission layer an electron transport layer stack is formed, preferably an electron transport layer is formed on the emission layer and a second electron transport layer is formed on the electron transport layer and the second electron transport layer may comprise the compound of Formula I and a metal dopant according to the invention,
- and finally, a cathode electrode is formed,
- optional a hole transport layer, an electron blocking layer formed in that order between the anode electrode and the emission layer,
- optional an electron injection layer is formed between the electron transport layer stack and the cathode electrode.

According to various embodiments of the present invention, the method may further include forming an electron injection layer on an electron transport layer. However, according to various embodiments of the OLED of the present invention, the OLED may not comprise an electron injection layer.

According to another aspect of the invention, it is provided an electronic device comprising at least one organic light emitting device according to any embodiment described throughout this application, preferably, the electronic device may comprise the organic light emitting diode in one of embodiments described throughout this application. More preferably, the electronic device is a display device.

### Definitions

The term "L is linkeded" means that Ar¹ and Ar² are bonded to L separated by one carbon atom on a benzene ring.

The term "organic metal complex" means a compound which comprises one or more metal and one or more organic groups. The metal may be bound to the organic group via a covalent or ionic bond. The organic group means a group comprising mainly covalently bound carbon and hydrogen atoms. The organic group may further comprise heteroatoms selected from N, O, S, B, Si, P, Se, preferably from B, N, O and S.

In the context of the present specification the term "essentially non-emissive" or "non-emitting" means that the visible emission spectrum from the composition or a layer of the compound of Formula I and at least one metal dopant. The visible emission spectrum is an emission spectrum with a wavelength of ≥ 380 nm to ≤ 780 nm. Preferably, an organic semiconductor layer or a device comprising a layer, which comprises the compound of Formula I and at least one a metal selected from Li, Na, Cs, Mg, Ca, Sr, Sm or Yb preferably Li, Cs, Mg or Yb and more preferably Li and Yb.

The term "free of", "does not contain", "does not comprise" does not exclude impurities which may be present in the compounds prior to deposition. Impurities have no technical effect with respect to the object achieved by the present invention.

The operating voltage, also named U, is measured in Volt (V) at 10 milliAmpere per square centimeter (mA/cm2).

The candela per Ampere efficiency, also named cd/A efficiency, is measured in candela per ampere at 10 milliAmpere per square centimeter (mA/cm2).

The external quantum efficiency, also named EQE, is measured in percent (%).

The color space is described by coordinates CIE-x and CIE-y (International Commission on Illumination 1931). For blue emission the CIE-y is of particular importance. A smaller CIE-y denotes a deeper blue color.

The highest occupied molecular orbital, also named HOMO, and lowest unoccupied molecular orbital, also named LUMO, are measured in electron volt (eV).

The rate onset temperature is measured in °C and describes the VTE source temperature at which measurable evaporation of a compound commences at a pressure of less than 10⁻⁵ mbar.

The term "OLED", "organic light emitting diode", "organic light emitting device", "organic optoelectronic device" and "organic light-emitting diode" are simultaneously used and have the same meaning.

The term "transition metal" means and comprises any element in the d-block of the periodic table, which comprises groups 3 to 12 elements on the periodic table.

The term "group III to VI metal" means and comprises any metal in groups III to VI of the periodic table.

As used herein, "weight percent", "wt.-%", "percent by weight", "% by weight", and variations thereof refer to a composition, component, substance or agent as the weight of that composition, component, substance or agent of the respective electron transport layer divided by the total weight of the composition thereof and multiplied by 100. It is understood that the total weight percent amount of all components, substances or agents of the respective electron transport layer are selected such that it does not exceed 100 wt.-%.

As used herein, "volume percent", "vol.-%", "percent by volume", "% by volume", and variations thereof refer to an elemental metal, a composition, component, substance or agent as the volume of that elemental metal, component, substance or agent of the respective electron transport layer divided by the total volume of the respective electron transport layer thereof and multiplied by 100. It is understood that the total volume percent amount of all elemental metal, components, substances or agents of the respective cathode electrode layer are selected such that it does not exceed 100 vol.-%.

All numeric values are herein assumed to be modified by the term "", whether or not explicitly indicated. As used herein, the term "" refers to variation in the numerical quantity that can occur.

Whether or not modified by the term "", the claims include equivalents to the quantities.

It should be noted that, as used in this specification and the appended claims, the singular forms "a", "an", and "the" include plural referents unless the content clearly dictates otherwise.

It should be noted that, as used in this specification and the appended claims, "*" if not otherwise defined indicates the chemical bonding position.

The anode electrode and cathode electrode may be described as anode electrode / cathode electrode or anode electrode / cathode electrode or anode electrode layer / cathode electrode layer.

In the present specification, when a definition is not otherwise provided, an "alkyl group" may refer to an aliphatic hydrocarbon group. The alkyl group may refer to "a saturated alkyl group" without any double bond or triple bond. The alkyl group may be a linear, cyclic or branched alkyl group.

The alkyl group may be a C₁ to C₁₆ alkyl group, or preferably a C₁ to C₁₂ alkyl group. More specifically, the alkyl group may be a C₁ to C₁₄ alkyl group, or preferably a C₁ to C₁₀ alkyl group or a C₁ to C₆ alkyl group. For example, a C₁ to C₄ alkyl group comprises 1 to 4 carbons in alkyl chain, and may be selected from methyl, ethyl, propyl, iso-propyl, n-butyl, iso-butyl, sec-butyl, and t-butyl.

Specific examples of the alkyl group may be a methyl group, an ethyl group, a propyl group, an isopropyl group, a butyl group, an isobutyl group, a tert-butyl group, a pentyl group, a hexyl group, a cyclopropyl group, a cyclobutyl group, a cyclopentyl group, a cyclohexyl group, and the like.

In the present specification, "aryl" and "arylene group" may refer to a group comprising at least one hydrocarbon aromatic moiety, and all the elements of the hydrocarbon aromatic moiety may have p-orbitals which form conjugation, for example a phenyl group, a naphthyl group, an anthracenyl group, a phenanthrenyl group, a pyrenyl group, a fluorenyl group and the like.

The term "heteroaryl" and "heteroarylene" may refer to aromatic heterocycles with at least one heteroatom, and all the elements of the aromatic heterocycle may have p-orbitals which form conjugation, for example a pyridyl, pyrimidyl, pyrazinyl, triazinyl, pyrrolyl, carbazolyl, furanyl, benzofuranyl, dibenzofuranyl, thiophenyl, benzothiophenyl, dibenzothiophenyl group and the like. Preferably, the aromatic heterocycles are free of sp³-hybridised carbon atoms.

The term "substituted or unsubstituted heteroaryl", "substituted or unsubstituted C₅ to C₂₄ heteroaryl", "substituted or unsubstituted C₃ to C₂₄ heteroaryl", "substituted or unsubstituted C₅ to C₁₈ heteroaryl", "substituted or unsubstituted C₅ to C₁₇ heteroaryl", "substituted or unsubstituted C₃ to C₁₇ heteroaryl", "substituted or unsubstituted C₃ to C₁₂ heteroarylene" and the like means that the substituted or unsubstituted heteroaryl comprises at least one heteroaryl ring; or at least one heteroaryl ring and at least one non-heteroaryl ring; or at least two heteroaryl rings and at least one non-heteroaryl ring; or at least three heteroaryl rings and at least one non-heteroaryl ring; or at least one heteroaryl ring and at least two non-heteroaryl rings. The rings of the substituted or unsubstituted heteroaryl can be a fused.

The term "hetero-fluorene ring" refers to a dibenzo[d,d]furanyl, dibenzo[b,d]thiophenyl or dibenzo[b,d]selenophenyl group.

The heteroatom may be selected from N, O, S, B, Si, P, Se, preferably from N, O and S.

A heteroarylene ring may comprise at least 1 to 3 heteroatoms. Preferably a heteroarylene ring may comprise at least 1 to 3 heteroatoms individually selected from N, S and/or O.

Further preferred in addition to the compounds of Formula I at least one additional heteroaryl/ene ring may comprise at least 1 to 3 N-atoms, or at least 1 to 2-N atoms or at least one N-atom.

Further preferred in addition to the compounds of Formula I at least one additional heteroaryl/ene ring may comprise at least 1 to 3 O-atoms, or at least 1 to 2 O-atoms or at least one O-atom.

Further preferred in addition to the compounds of Formula I at least one additional heteroaryl/ene ring may comprise at least 1 to 3 S-atoms, or at least 1 to 2 S-atoms or at least one S-atom.

According to another preferred embodiment, wherein the compound according to Formula I may comprise:
- at least 9 to 25 aromatic rings, preferably at least 9 to 22 aromatic rings, further preferred at least 9 to 20 aromatic rings, in addition preferred at least 9 to 15 aromatic rings and more preferred at least 10 to 14 aromatic rings; wherein
- at least 2 to 5, preferably 3 to 4 or 2 to 3 are heteroaromatic rings.

According to one embodiment, wherein the compound according to Formula I:
- comprises at least 9 to 20 aromatic rings, preferably at least 9 to 18 aromatic rings, further preferred at least 9 to 16 aromatic rings, in addition preferred at least 10 to 15 aromatic rings and more preferred at least 11 to 14 aromatic rings; and/or
- the compound of Formula I comprises at least 2 to 6, preferably 3 to 5 or 2 to 4, hetero aromatic rings, wherein the hetero atoms can be selected from N, O, S.

According to one embodiment, wherein the compound according to Formula I can be free of a fluorene ring and free of a hetero-fluorene ring.

According to one embodiment, wherein the compound according to Formula I can be free of a spiro-group.

According to a further preferred embodiment, wherein the compound of Formula I comprises at least 2 to 7, preferably 2 to 5, or 2 to 3 hetero aromatic rings.

According to a further preferred embodiment, wherein the compound of Formula I comprises at least 2 to 7, preferably 2 to 5, or 2 to 3 hetero aromatic rings, wherein at least one of the aromatic rings is a five-member hetero aromatic ring.

According to a further preferred embodiment, wherein the compound of Formula I comprises at least 3 to 7, preferably 3 to 6, or 3 to 5 hetero aromatic rings, wherein at least two of the hetero aromatic rings are five member hetero-aromatic-rings.

According to one embodiment, wherein the compound according to Formula I may comprise at least 6 to 12 non-hetero aromatic rings and 2 to 3 hetero aromatic rings.

According to one preferred embodiment, wherein the compound according to Formula I may comprise at least 7 to 12 non-hetero aromatic rings and 2 to 5 hetero aromatic rings.

According to one preferred embodiment, wherein the compound according to Formula I may comprise at least 7 to 11 non-hetero aromatic rings and 2 to 3 hetero aromatic rings.

### Melting point

The melting point (mp) is determined as peak temperatures from the DSC curves of the above TGA-DSC measurement or from separate DSC measurements (Mettler Toledo DSC822e, heating of samples from room temperature to completeness of melting with heating rate 10 K/min under a stream of pure nitrogen. Sample amounts of 4 to 6 mg are placed in a 40 µL Mettler Toledo aluminum pan with lid, a <1 mm hole is pierced into the lid).

According to another embodiment, wherein the compound of Formula I may have a melting point of ≥ 220° C and ≤ 380° C, preferably ≥ 260° C and ≤ 370° C, further preferred ≥ 265° C and ≤ 360° C.

### Glass transition temperature

The glass transition temperature is measured under nitrogen and using a heating rate of 10 K per min in a Mettler Toledo DSC 822e differential scanning calorimeter as described in DIN EN ISO 11357, published in March 2010.

According to another embodiment, wherein the compound of Formula I may have a glass transition temperature Tg of ≥ 105° C and ≤ 380° C, preferably ≥ 110° C and ≤ 350° C, and further preferred ≥ 130° C and ≤ 180° C.

### Rate onset temperature

The rate onset temperature is determined by loading 100 mg compound into a VTE source. As VTE source a point source for organic materials is used as supplied by Kurt J. Lesker Company (www.lesker.com) or CreaPhys GmbH (www.creaphys.com). The VTE source is heated at a constant rate of 15 K/min at a pressure of less than 10⁻⁵ mbar and the temperature inside the source measured with a thermocouple. Evaporation of the compound is detected with a QCM detector which detects deposition of the compound on the quartz crystal of the detector. The deposition rate on the quartz crystal is measured in Ǻngstrom per second. To determine the rate onset temperature, the deposition rate is plotted against the VTE source temperature. The rate onset is the temperature at which noticeable deposition on the QCM detector occurs. For accurate results, the VTE source is heated and cooled three time and only results from the second and third run are used to determine the rate onset temperature.

To achieve good control over the evaporation rate of an organic compound, the rate onset temperature may be in the range of 200 to 255 °C. If the rate onset temperature is below 200 °C the evaporation may be too rapid and therefore difficult to control. If the rate onset temperature is above 255 °C the evaporation rate may be too low which may result in low takt time and decomposition of the organic compound in VTE source may occur due to prolonged exposure to elevated temperatures.

The rate onset temperature is an indirect measure of the volatility of a compound. The higher the rate onset temperature the lower is the volatility of a compound.

According to another embodiment, wherein the compound of Formula I may have a rate onset temperature T_{RO} of ≥ 200° C and ≤ 260° C, preferably ≥ 220° C and ≤ 260° C, further preferred ≥ 220° C and ≤ 260° C, in addition preferred ≥ 230° C and ≤ 255° C.

### Dipole moment

The dipole moment |*̅µ̅*̅| of a molecule containing N atoms is given by: $\vec{μ} = {\sum }_{i}^{N} {q}_{i} \vec{{r}_{ι}}$ $\left|\vec{μ}\right| = \sqrt{{μ}_{x}^{2} + {μ}_{y}^{2} + {μ}_{z}^{2}}$ where *qᵢ* and *̅r̅ᵢ̅*̅ are the partial charge and position of atom i in the molecule.

The dipole moment is determined by a semi-empirical molecular orbital method. The geometries of the molecular structures are optimized using the hybrid functional B3LYP with the 6-31G* basis set in the gas phase as implemented in the program package TURBOMOLE V6.5 (TURBOMOLE GmbH, Litzenhardtstrasse 19, 76135 Karlsruhe, Germany). If more than one conformation is viable, the conformation with the lowest total energy may be selected to determine the bond lengths of the molecules.

According to one embodiment the compounds according to Formula I may have a dipole moment (Debye) in the range from ≥ 1 to ≤ 6, preferably from ≥ 2 to ≤ 5.5, further preferred from ≥ 3 to ≤ 5 and additional preferred from ≥ 3.5 to ≤ 4.8.

### Calculated HOMO and LUMO

The HOMO and LUMO are calculated with the program package TURBOMOLE V6.5. The optimized geometries and the HOMO and LUMO energy levels of the molecular structures are determined by applying the hybrid functional B3LYP with a 6-31G* basis set in the gas phase. If more than one conformation is viable, the conformation with the lowest total energy is selected.

According to one embodiment the compounds according to Formula I may have a LUMO energy level (eV) in the range from - 2.20 eV to - 1.50 eV, preferably from - 2.0 eV to - 1.55 eV, further preferred from - 1.9 eV to - 1.6 eV, also preferred from - 1.8 eV to - 1.7 eV.

### Technical effect

Surprisingly, it was found that the organic electronic device comprising organic semiconductor layer comprising compound of formula 1 according to the present invention solve the problem underlying the present invention by being superior over the organic electronic device known in the art, in particular with respect to operating voltage, which is important for reducing power consumption and increasing battery life, for example of a mobile display device. At the same time the cd/A efficiency, also referred to as current efficiency is kept at a similar or even improved level. Long life time at high current density is important for the longevity of a device which run at high brightness.

Likewise, some compounds represented by Formula I falling within the scope of the broadest definition of the present invention have surprisingly be found to be particularly well performing with respect to the mentioned property of glass transition temperature, rate onset temperature and/ or operating voltage in organic electronic devices. These compounds are discussed herein to be particularly preferred.

The beneficial effect of the invention on the performance of organic electronic devices can be seen in Table 1 and Table 2.

### Anode

A material for the anode may be a metal or a metal oxide, or an organic material, preferably a material with work function above 4.8 eV, more preferably above 5.1 eV, most preferably above 5.3 eV. Preferred metals are noble metals like Pt, Au or Ag, preferred metal oxides are transparent metal oxides like ITO or IZO which may be advantageously used in bottom-emitting OLEDs having a reflective cathode.

In devices comprising a transparent metal oxide anode or a reflective metal anode, the anode may have a thickness from 50 nm to 100 nm, whereas semitransparent metal anodes may be as thin as from 5 nm to 15 nm, and non-transparent metal anodes may have a thickness from 15 nm to 150nm.

### Hole injection layer (HIL)

The hole injection layer may improve interface properties between the anode and an organic material used for the hole transport layer, and is applied on a non-planarized anode and thus may planarize the surface of the anode. For example, the hole injection layer may include a material having a median value of the energy level of its highest occupied molecular orbital (HOMO) between the work function of the anode material and the energy level of the HOMO of the hole transport layer, in order to adjust a difference between the work function of the anode and the energy level of the HOMO of the hole transport layer.

When the hole transport region comprises a hole injection layer, the hole injection layer may be formed on the anode by any of a variety of methods, for example, vacuum deposition, spin coating, casting, Langmuir-Blodgett (LB) method, or the like.

When hole injection layer is formed using vacuum deposition, vacuum deposition conditions may vary depending on the material that is used to form the hole injection layer, and the desired structure and thermal properties of the hole injection layer to be formed and for example, vacuum deposition may be performed at a temperature of 100 °C to 500 °C, a pressure of 10⁻⁶ Pa to 10⁻¹ Pa, and a deposition rate of 0.1 to 10 nm/sec, but the deposition conditions are not limited thereto.

When the hole injection layer is formed using spin coating, the coating conditions may vary depending on the material that is used to form the hole injection layer, and the desired structure and thermal properties of the hole injection layer to be formed. For example, the coating rate may be in the range of 2000 rpm to 5000 rpm, and a temperature at which heat treatment is performed to remove a solvent after coating may be in a range of 80 °C to 200 °C, but the coating conditions are not limited thereto.

According to another embodiment the hole injection layer comprises at least one radialene compound, wherein the radialene compound is a [3]-radialene, and preferably the radialene compound is a [3]-radialene and selected from the group A1 to A21
The hole injection layer may further comprise a p-dopant to improve conductivity and/or hole injection from the anode.

### p-dopant

In another aspect, the p-dopant may be homogeneously dispersed in the hole injection layer.

In another aspect, the p-dopant may be present in the hole injection layer in a higher concentration closer to the anode and in a lower concentration closer to the cathode.

The p-dopant may be one of a quinone derivative or a radialene compound but not limited thereto. Non-limiting examples of the p-dopant are quinone derivatives such as tetracyanoquinonedimethane (TCNQ), 2,3,5,6-tetrafluoro-tetracyano-1,4-benzoquinonedimethane (F4-TCNQ), 4,4',4"-((1E,1'E,1"E)-cyclopropane-1,2,3-triylidenetris(cyanomethanylylidene))-tris(2,3,5,6-tetrafluorobenzonitrile).

According to another embodiment, an organic electronic device comprising an organic semiconductor layer comprising a composition according to invention may additional comprise a layer comprising a radialene compound and/or a quinodimethane compound.

In another embodiment, the radialene compound and/or the quinodimethane compound may be substituted with one or more halogen atoms and/or with one or more electron withdrawing groups. Electron withdrawing groups can be selected from nitrile groups, halogenated alkyl groups, alternatively from perhalogenated alkyl groups, alternatively from perfluorinated alkyl groups. Other examples of electron withdrawing groups may be acyl, sulfonyl groups or phosphoryl groups.

Alternatively, acyl groups, sulfonyl groups and/or phosphoryl groups may comprise halogenated and/or perhalogenated hydrocarbyl. In one embodiment, the perhalogenated hydrocarbyl may be a perfluorinated hydrocarbyl. Examples of a perfluorinated hydrocarbyl can be perfluormethyl, perfluorethyl, perfluorpropyl, perfluorisopropyl, perfluorobutyl, perfluorophenyl, perfluorotolyl; examples of sulfonyl groups comprising a halogenated hydrocarbyl may be trifluoromethylsulfonyl, pentafluoroethylsulfonyl, pentafluorophenylsulfonyl, heptafluoropropylsufonyl, nonafluorobutylsulfonyl, and like.

In one embodiment, the radialene and/or the quinodimethane compound may be comprised in a hole injection, hole transporting and/or a hole generation layer.

In one embodiment, the radialene compound may have formula (XX) and/or the quinodimethane compound may have formula (XXIa) or (XXIb): 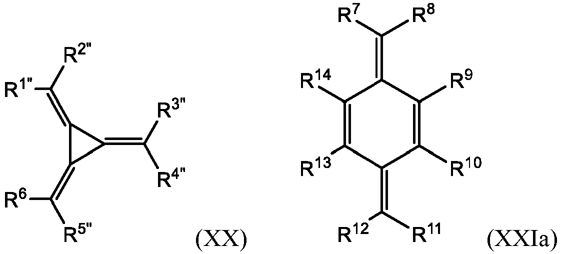 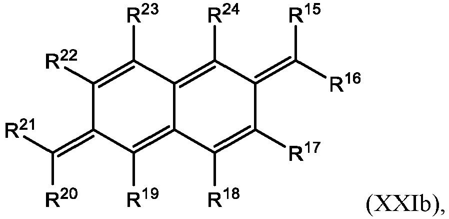 wherein R^{1"}, R^{2"}, R^{3"}, R^{4"}, R^{5"}, R⁶, R⁷, R⁸, R¹¹, R¹², R¹⁵, R¹⁶, R²⁰, R²¹ may be independently selected from an electron withdrawing groups and R⁹, R¹⁰, R¹³, R¹⁴, R¹⁷, R¹⁸, R¹⁹, R²², R²³ and R²⁴ may be independently selected from H, halogen and electron withdrawing groups. Electron withdrawing group/s that can be suitable used are above mentioned.

### Hole transport layer (HTL)

Conditions for forming the hole transport layer and the electron blocking layer may be defined based on the above-described formation conditions for the hole injection layer.

A thickness of the hole transport part of the charge transport region may be from 10 nm to 1000 nm, for example, 10 nm to 100 nm. When the hole transport part of the charge transport region comprises the hole injection layer and the hole transport layer, a thickness of the hole injection layer may be from 10 nm to 1000 nm, for example 10 nm to 100 nm and a thickness of the hole transport layer may be from 5 nm to 200 nm, for example 10 nm to 150 nm. When the thicknesses of the hole transport part of the charge transport region, the HIL, and the HTL are within these ranges, satisfactory hole transport characteristics may be obtained without a substantial increase in operating voltage.

Hole transport matrix materials used in the hole transport region are not particularly limited. Preferred are covalent compounds comprising a conjugated system of at least 6 delocalized electrons, preferably organic compounds comprising at least one aromatic ring, more preferably organic compounds comprising at least two aromatic rings, even more preferably organic compounds comprising at least three aromatic rings, most preferably organic compounds comprising at least four aromatic rings. Typical examples of hole transport matrix materials which are widely used in hole transport layers are polycyclic aromatic hydrocarbons, triarylene amine compounds and heterocyclic aromatic compounds. Suitable ranges of frontier orbital energy levels of hole transport matrices useful in various layer of the hole transport region are well-known. In terms of the redox potential of the redox couple HTL matrix/ cation radical of the HTL matrix, the preferred values (if measured for example by cyclic voltammetry against ferrocene/ferrocenium redox couple as reference) may be in the range 0.0 - 1.0 V, more preferably in the range 0.2 - 0.7 V, even more preferably in the range 0.3 - 0.5 V.

### Buffer layer

The hole transport part of the charge transport region may further include a buffer layer.

Buffer layer that can be suitable used are disclosed in US 6 140 763, US 6 614 176 and in US2016/248022.

The buffer layer may compensate for an optical resonance distance of light according to a wavelength of the light emitted from the EML, and thus may increase efficiency.

### Emission layer (EML)

The emission layer may be formed on the hole transport region by using vacuum deposition, spin coating, casting, LB method, or the like. When the emission layer is formed using vacuum deposition or spin coating, the conditions for deposition and coating may be similar to those for the formation of the hole injection layer, though the conditions for the deposition and coating may vary depending on the material that is used to form the emission layer. The emission layer may include an emitter host (EML host) and an emitter dopant (further only emitter).

A thickness of the emission layer may be 100Å to 1000Å, for example 200Å to 600Å. When the thickness of the emission layer is within these ranges, the emission layer may have improved emission characteristics without a substantial increase in operating voltage.

### Emitter host

According to another embodiment, the emission layer may comprise compound of Formula I as emitter host.

The emitter host compound has at least three aromatic rings, which may be independently selected from carbocyclic rings and heterocyclic rings.

Other compounds that can be used as the emitter host is an anthracene matrix compound represented by formula 400 below:

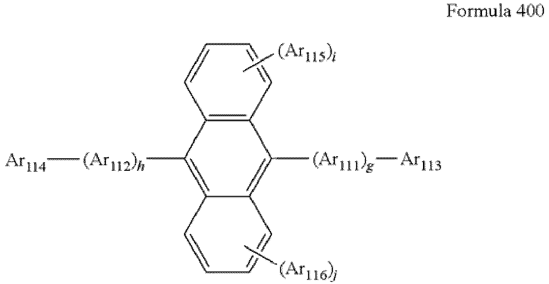

In formula 400, Ar₁₁₁ and Ar₁₁₂ may be each independently a substituted or unsubstituted C₆-C₆₀ arylene group; Ar₁₁₃ to Ar₁₁₆ may be each independently a substituted or unsubstituted C₁-C₁₀ alkyl group or a substituted or unsubstituted C₆-C₆₀ arylene group; and g, h, i, and j may be each independently an integer from 0 to 4.

In some embodiments, Ar₁₁₁ and Ar₁₁₂ in formula 400 may be each independently one of a phenylene group, a naphthalene group, a phenanthrenylene group, or a pyrenylene group; or
a phenylene group, a naphthalene group, a phenanthrenylene group, a fluorenyl group, or a pyrenylene group, each substituted with at least one of a phenyl group, a naphthyl group, or an anthryl group.

In formula 400, g, h, i, and j may be each independently an integer of 0, 1, or 2.

In formula 400, Ar₁₁₃ to Ar₁₁₆ may be each independently one of
- a C₁-C₁₀ alkyl group substituted with at least one of a phenyl group, a naphthyl group, or an anthryl group;
- a phenyl group, a naphthyl group, an anthryl group, a pyrenyl group, a phenanthrenyl group, or a fluorenyl group;
- a phenyl group, a naphthyl group, an anthryl group, a pyrenyl group, a phenanthrenyl group, or a fluorenyl group, each substituted with at least one of a deuterium atom, a halogen atom, a hydroxyl group, a cyano group, a nitro group, an amino group, an amidino group, a hydrazine group, a hydrazone group, a carboxyl group or a salt thereof,
- a sulfonic acid group or a salt thereof, a phosphoric acid group or a salt thereof,
- a C₁-C₆₀ alkyl group, a C₂-C₆₀ alkenyl group, a C₂-C₆₀ alkynyl group, a C₁-C₆₀ alkoxy group, a phenyl group, a naphthyl group, an anthryl group, a pyrenyl group, a phenanthrenyl group, or
- a fluorenyl group 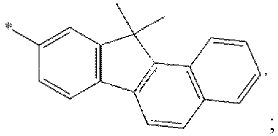 or
- formulas 7 or 8

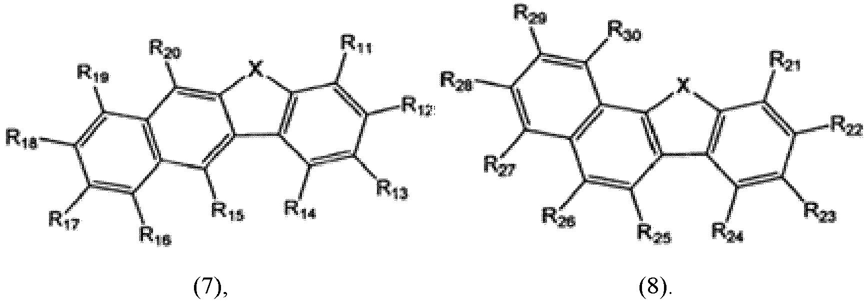

Wherein in the formulas 7 and 8, X may be selected form an oxygen atom and a sulfur atom, but embodiments of the invention are not limited thereto.

In the formula 7, any one of R₁₁ to R₁₄ is used for bonding to Ar₁₁₁. R₁₁ to R₁₄ that are not used for bonding to Ar₁₁₁ and R₁₅ to R₂₀ are the same as R₁ to R₈.

In the formula 8, any one of R₂₁ to R₂₄ is used for bonding to Ar₁₁₁. R₂₁ to R₂₄ that are not used for bonding to Ar₁₁₁ and R₂₅ to R₃₀ are the same as R₁ to R₈.

Preferably, the EML host comprises between one and three heteroatoms selected from the group consisting of N, O or S. More preferred the EML host comprises one heteroatom selected from S or O.

### Emitter dopant

The dopant is mixed in a small amount to cause light emission, and may be generally a material such as a metal complex that emits light by multiple excitation into a triplet or more. The dopant may be, for example an inorganic, organic, or organic/inorganic compound, and one or more kinds thereof may be used.

The emitter may be a red, green, or blue emitter.

The dopant may be a fluorescent dopant, for example ter-fluorene, the structures are shown below. 4.4'-bis(4-diphenyl amiostyryl)biphenyl (DPAVBI, 2,5,8,11-tetra-tert-butyl perylene (TBPe), and Compound 8 below are examples of fluorescent blue dopants.

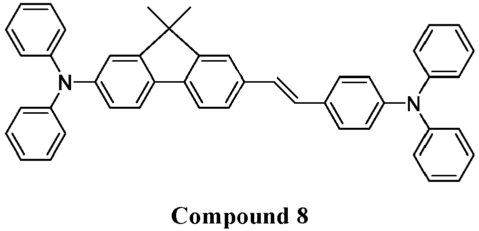

The dopant may be a phosphorescent dopant, and examples of the phosphorescent dopant may be an organic metal compound comprising Ir, Pt, Os, Ti, Zr, Hf, Eu, Tb, Tm, Fe, Co, Ni, Ru, Rh, Pd, or a combination thereof. The phosphorescent dopant may be, for example a compound represented by formula Z, but is not limited thereto:

J₂MX (Z).

In formula Z, M is a metal, and J and X are the same or different, and are a ligand to form a complex compound with M.

The M may be, for example Ir, Pt, Os, Ti, Zr, Hf, Eu, Tb, Tm, Fe, Co, Ni, Ru, Rh, Pd or a combination thereof, and the J and X may be, for example a bidendate ligand.

One or more emission layers may be arranged between the anode and the cathode. To increase overall performance, two or more emission layers may be present.

### Charge generation layer

A charge generation layer (also named CGL) may be arranged between the first emission layer and the second emission layer, and second and third emission layer, if present. Typically, the CGL comprises an n-type charge generation layer (also named n-CGL or electron generation layer) and a p-type charge generation layer (also named p-CGL or hole generation layer). An interlayer may be arranged between the n-type CGL and the p-type CGL.

According to another embodiment, the organic semiconductor layer that comprises the compound of Formula I is an n-type charge generation layer. In another embodiment the n-type charge generation layer may comprise the compound of Formula I according to the present invention.

In another aspect, the at least n-type charge generation layer may comprise a compound of Formula I and a dopant, wherein the dopant is selected from the group comprising a metal, metal salt or organic metal complex.

In another aspect, the at least n-type charge generation layer may comprise a compound of Formula I and a dopant, wherein the dopant is a metal selected from Li, Na, Cs, Mg, Ca, Sr, Sm or Yb preferably from Li, Cs, Mg or Yb.

. In another aspect, the at least n-type charge generation layer may comprise a compound of Formula I and a dopant, wherein the dopant is a metal selected preferably from Li or Yb.

### Electron transport layer (ETL)

According to another embodiment, the organic semiconductor layer that comprises the compound of Formula I is an electron transport layer. In another embodiment an electron transport layer may comprise the compound of Formula I.

In another aspect, the at least an electron transport layer may comprise a compound of Formula I and a dopant, wherein the dopant is selected from the group comprising a metal, metal salt or organic metal complex.

In another aspect, the at least an electron transport layer may comprise a compound of Formula I and a dopant, wherein the dopant is a metal selected from Li, Na, Cs, Mg, Ca, Sr, Sm or Yb preferably from Li, Cs, Mg or Yb.

In another aspect, the at least an electron transport layer may comprise a compound of Formula I and a dopant, wherein the dopant is a metal selected preferably from Li or Yb.

In another embodiment, the organic electronic device comprises an electron transport region of a stack of organic layers formed by two or more electron transport layers, wherein at least one electron transport layer comprises the compound of Formula I.

In another embodiment, the organic electronic device comprises an electron transport region of a stack of organic layers formed by two or more electron transport layers, wherein at least one electron transport layer may comprise the compound of Formula I and a dopant, wherein the dopant is selected from the group comprising a metal, metal salt or organic metal complex.

In another embodiment, the organic electronic device comprises an electron transport region of a stack of organic layers formed by two or more electron transport layers, wherein at least one electron transport layer may comprise the compound of Formula I and a dopant, wherein the dopant is a metal selected from Li, Na, Cs, Mg, Ca, Sr, Sm or Yb preferably from Li, Cs, Mg or Yb.

The thickness of the electron transport layer may be from 0.5 nm to 100 nm, for example 2 nm to 40 nm. When the thickness of the electron transport layer is within these ranges, the electron transport layer may have improved electron transport ability without a substantial increase in operating voltage.

### Electron injection layer (EIL)

According to another aspect of the invention, the organic electronic device may further comprise an electron injection layer between the electron transport layer (-ETL) and the cathode.

The electron injection layer (EIL) may facilitate injection of electrons from the cathode.

According to another aspect of the invention, the electron injection layer may comprise:
(i) a metal selected from alkali metals, alkaline earth metals and rare earth metals in substantially elemental form, preferably selected from Li, Na, K, Rb, Cs, Mg, Ca, Sr, Ba, Eu and Yb, more preferably from Li, Na, Mg, Ca, Sr and Yb, even more preferably from Li and Yb, most preferably Yb; and/or
(ii) an alkali metal complex and/or alkali metal salt, preferably the Li complex and/or salt, more preferably a Li quinolinolate, even more preferably a lithium 8-hydroxyquino-linolate.

The electron injection layer may include at least one selected from LiF, NaCl, CsF, Li₂O, and BaO.

A thickness of the EIL may be from 0.1 nm to 10 nm, or 0.3 nm to 9 nm. When the thickness of the electron injection layer is within these ranges, the electron injection layer may have satisfactory electron injection ability without a substantial increase in operating voltage.

The electron injection layer may comprise or consist of the compound of Formula I and a metal dopant according to the invention.

### Cathode

A material for the cathode may be a metal, an alloy, or an electrically conductive compound that have a low work function, or a combination thereof. Specific examples of the material for the cathode may be lithium (Li), magnesium (Mg), aluminum (Al), aluminum-lithium (Al-Li), calcium (Ca), magnesium-indium (Mg-In), magnesium-silver (Mg-Ag), silver (Ag) etc. In order to manufacture a top-emission light-emitting device having a reflective anode deposited on a substrate, the cathode may be formed as a light-transmissive electrode from, for example, indium tin oxide (ITO), indium zinc oxide (IZO) or silver (Ag).

In devices comprising a transparent metal oxide cathode or a reflective metal cathode, the cathode may have a thickness from 50 nm to 100 nm, whereas semitransparent metal cathodes may be as thin as from 5 nm to 15 nm.

### Substrate

A substrate may be further disposed under the anode or on the cathode. The substrate may be a substrate that is used in a general organic light emitting diode and may be a glass substrate or a transparent plastic substrate with strong mechanical strength, thermal stability, transparency, surface smoothness, ease of handling, and water resistance.

Hereinafter, the embodiments are illustrated in more detail with reference to examples. However, the present disclosure is not limited to the following examples.

### BRIEF DESCRIPTION OF THE DRAWINGS

These and/or other aspects and advantages of the present invention will become apparent and more readily appreciated from the following description of the exemplary embodiments, taken in conjunction with the accompanying drawings, of which:
- FIG. 1: is a schematic sectional view of an organic light-emitting diode (OLED), according to an exemplary embodiment of the present invention;
- FIG. 2: is a schematic sectional view of an OLED, according to an exemplary embodiment of the present invention.
- FIG. 3: is a schematic sectional view of a tandem OLED comprising a charge generation layer, according to an exemplary embodiment of the present invention.
- FIG. 4: is a schematic sectional view of a tandem OLED comprising a charge generation layer, according to an exemplary embodiment of the present invention.

### DETAILED DESCRIPTION

Reference will now be made in detail to the exemplary embodiments of the present invention, examples of which are illustrated in the accompanying drawings, wherein like reference numerals refer to the like elements throughout. The exemplary embodiments are described below, in order to explain the aspects of the present invention, by referring to the figures.

Herein, when an element is referred to as being formed or disposed "on" a second element, the element can be disposed directly on the second element, or one or more other elements may be disposed there between. When an element is referred to as being formed or disposed "directly on" a second element, no other elements are disposed there between.

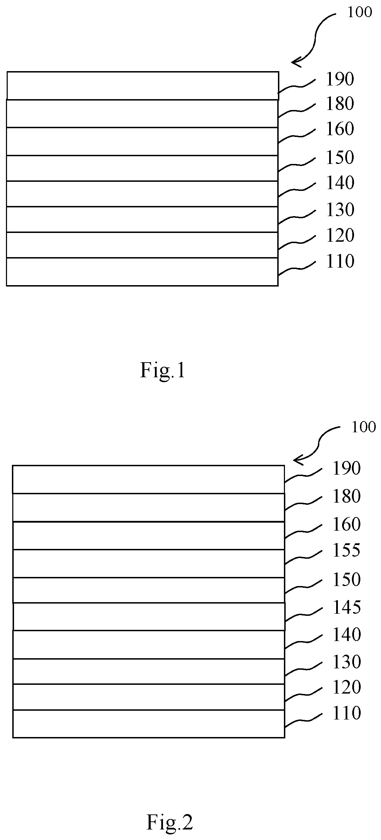

FIG. 1 is a schematic sectional view of an organic light-emitting diode (OLED) 100, according to an exemplary embodiment of the present invention. The OLED 100 includes a substrate 110, an anode 120, a hole injection layer (HIL) 130, a hole transport layer (HTL) 140, an emission layer (EML) 150, an electron transport layer (ETL) 160, an electron injection layer (EIL) 180 and a cathode 190. The electron transport layer (ETL) 160 comprises or consists of the compound of Formula I and optional a dopant.

Instead of a single electron transport layer 160, optional an electron transport layer stack (ETL) can be used. The electron transport layer stack (ETL) comprises a first electron transport layer and a second electron transport layer, wherein the first electron transport layer is arranged near to EML and the second electron transport layer is arranged near to the cathode (190). The first and/or the second electron transport layer comprise the compound of Formula I according to the invention and optional a dopant according to the invention.

Fig. 2 is a schematic sectional view of an OLED 100, according to another exemplary embodiment of the present invention. Fig. 2 differs from Fig. 1 in that the OLED 100 of Fig. 2 comprises an electron blocking layer (EBL) 145 and a hole blocking layer (HBL) 155. Referring to Fig. 2 the OLED 100 includes a substrate 110, an anode 120, a hole injection layer (HIL) 130, a hole transport layer (HTL) 140, an electron blocking layer (EBL) 145, an emission layer (EML) 150, a hole blocking layer (HBL) 155, an electron transport layer (ETL) 160 and a second electron transport layer (ETL) 161, an electron injection layer (EIL) 180 and a cathode electrode 190. The electron transport layer (ETL) 160 and/or the electron injection layer (EIL) 180 comprise or consist of the compound of Formula I and optional a dopant.

In the description above the method of manufacture an OLED 100 of the present invention is started with a substrate 110 onto which an anode 120 is formed, on the anode electrode 120, an hole injection layer 130, hole transport layer 140, optional an electron blocking layer 145, an emission layer 150, optional a hole blocking layer 155, at least a first electron transport layer 160 and optional at least one second electron transport layer 161, optional at least one electron injection layer 180, and a cathode 190 are formed, in that order or the other way around.

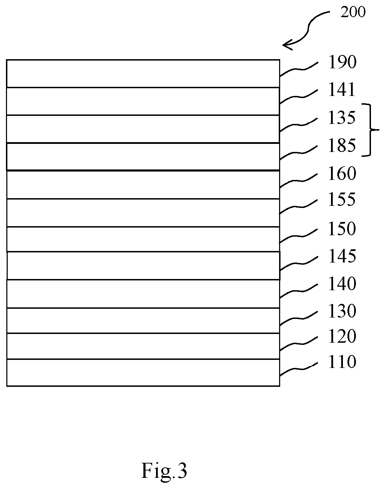

Fig. 3 is a schematic sectional view of a tandem OLED 200, according to another exemplary embodiment of the present invention. Fig. 3 differs from Fig. 2 in that the OLED 100 of Fig. 3 further comprises a charge generation layer and a hole transport layer (HTL) 141.

Referring to Fig. 3 the OLED 200 includes a substrate 110, an anode 120, a hole injection layer (HIL) 130, a hole transport layer (HTL) 140, an electron blocking layer (EBL) 145, a emission layer (EML) 150, a hole blocking layer (HBL) 155, a first electron transport layer (ETL) 160, a second electron transport layer (ETL) 161, an n-type charge generation layer (n-type CGL) 185, a p-type charge generation layer (p-type GCL) 135, a second hole transport layer (HTL) 141 and a cathode 190. The first electron transport layers (ETL) 160 or the second electron transport layers (ETL) 161, and the electron injection layer (EIL) 180 and/or the n-type charge generation layer (n-type CGL) 185 comprise or consist of the compound of Formula I and optional a metal dopant.

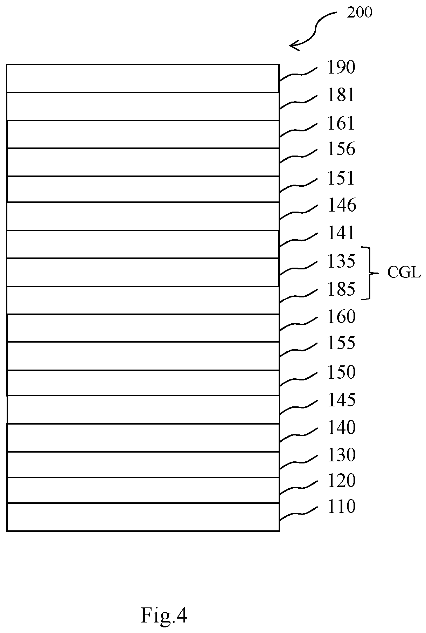

Fig. 4 is a schematic sectional view of a tandem OLED 200, according to another exemplary embodiment of the present invention. Fig. 4 differs from Fig. 2 in that the OLED 100 of Fig. 3 further comprises a charge generation layer and a second emission layer. Referring to Fig. 4 the OLED 200 includes a substrate 110, an anode 120, a hole injection layer (HIL) 130, a hole transport layer (HTL) 140, an electron blocking layer (EBL) 145, a emission layer (EML) 150, a hole blocking layer (HBL) 155, an electron transport layer (ETL) 160, an n-type charge generation layer (n-type CGL) 185 which may comprise compound of Formula I and optional a metal dopant, a p-type charge generation layer (p-type GCL) 135, a second hole transport layer (HTL) 141, a second electron blocking layer (EBL) 146, a second emission layer (EML) 151, a second hole blocking layer (EBL) 156, a second electron transport layer (ETL) 161, a second electron injection layer (EIL) 181 and a cathode 190. The electron transport layers (ETL) 160 and optional the electron injection layer (EIL) 180 and/or the n-type charge generation layer (n-type CGL) 185 comprise or consist of the compound of Formula I and optional a dopant. In the description above the method of manufacture an OLED 200 of the present invention is started with a substrate 110 onto which an anode 120 is formed, on the anode electrode 120, a hole injection layer 130, hole transport layer 140, optional an electron blocking layer 145, a emission layer 150, optional a hole blocking layer 155, optional at least one electron transport layer 160, an n-type CGL 185, a p-type CGL 135, a second hole transport layer 141, optional a second electron blocking layer 146, a second emission layer 151, an optional second hole blocking layer 156, an at least one second electron transport layer 161, an optional second electron injection layer (EIL) 181 and a cathode 190 are formed, in that order or the other way around.

Hereinafter, the embodiments are illustrated in more detail with reference to examples. However, the present disclosure is not limited to the following examples. Reference will now be made in detail to the exemplary aspects.

### Preparation of compounds of Formula I

### General procedure for the synthesis of compound of formula 1

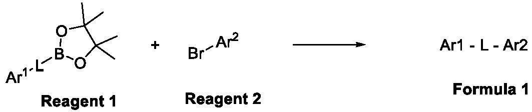

A reactor was flushed with nitrogen and charged with reagent 1 (1eq), reagent 2 and potassium carbonate ( 3eq). 1,4-Dioxane (1500 mL) and water (250mL) were added and the reaction mixture was degassed with N2 for 20 min. The first portion of the catalyst [1,1'-bis(diphenylphosphino)ferrocene]dichloropalladium(II) was added (0.01eq). The reaction mixture was heated to 100°C and stirred for 72 h. Then a second portion of the catalyst was added (0.01eq) and stirring was continued for 100 h at 100°C.

The reaction mixture was allowed to cool to room temperature. The product precipitated and was collected by filtration. The solid material was washed with water (2 x 500mL) and dried under vacuum at 60°C overnight. The raw product was purified by hot filtration over 2cm silica gel. 8L of chlorobenzene were used for dissolving the product and further 6L enriched with methanol (60mL) for eluting it. The solution was concentrated to 5L, stirred overnight and the precipitate was collected by filtration. The solid material was washed with chlorobenzene (2 x 300mL) and dried at 60°C under vacuum overnight. The product was recrystallized from DMF.

### Synthesis of 7-(3-(9-phenyl-1,10-phenanthrolin-2-yl)phenyl)dibenzo[c,h]acridine (G1):

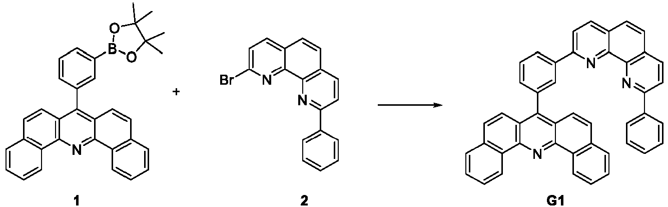

A 3-neck round bottom flask was flushed with nitrogen and charged with 7-(3-(4,4,5,5-tetramethyl-1,3,2-dioxaborolan-2-yl)phenyl)dibenzo[c,h]acridine (1) (8.28g, 17.2mmol, 1eq), 2-chloro-9-phenyl-1,10-phenanthroline (2) (5.00g, 17.2mmol, 1eq), potassium carbonate (7.13g, 51.6mmol, 3eq), 1,4-dioxane (156 mL) and water (26 mL). The reaction mixture was degassed with N2 for 30 min and the catalyst [1,1'-bis(diphenylphosphino)ferrocene]dichloropalladium(II) (0.38g, 0.52mmol, 0.03eq) was added. The mixture was stirred for 24h at 95°C. The reaction mixture was allowed to cool to room temperature and the product was collected by filtration. The solid material was washed with water until it was neutral and was dried at 70°C under vacuum overnight. The raw product was dissolved in DCM, filtered over AloxN and the solvent was removed. The product was recrystallized from chlorobenzene. 40% yield, 99.85% HPLC purity at 292nm. Further purification was achieved by high vacuum sublimation with 89% yield. ESI-MS 610 (M+H).

### Synthesis of 7-(3-(1,10-phenanthrolin-2-yl)phenyl)dibenzo[c,h]acridine (G2):

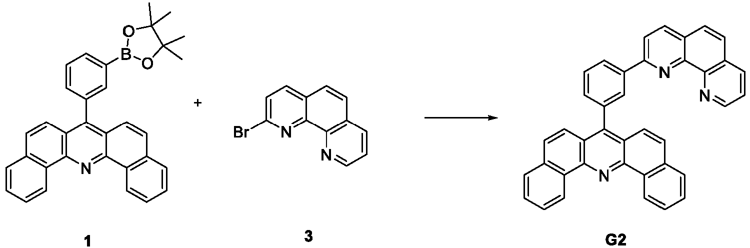

A reactor was flushed with nitrogen and charged with 7-(3-(4,4,5,5-tetramethyl-1,3,2-dioxaborolan-2-yl)phenyl)dibenzo[c,h]acridine (1) , 2-bromo-1,10-phenanthroline (3) (100.0g, 0.386mol, 1eq) and potassium carbonate (160g, 1.16mol, 3eq). 1,4-Dioxane (1500 mL) and water (250mL) were added and the reaction mixture was degassed with N2 for 20 min. The first portion of the catalyst [1,1'-bis(diphenylphosphino)ferrocene]dichloropalladium(II) was added (2.8g, 3.86mmol, 0.01eq). The reaction mixture was heated to 100°C and stirred for 72 h. Then a second portion of the catalyst was added (2.8g, 3.86mmol, 0.01eq) and stirring was continued for 100 h at 100°C.The reaction mixture was allowed to cool to room temperature. The product precipitated and was collected by filtration. The solid material was washed with water (2 x 500mL) and dried under vacuum at 60°C overnight. The raw product was purified by hot filtration over 2cm silica gel. 8L of chlorobenzene were used for dissolving the product and further 6L enriched with methanol (60mL) for eluting it. The solution was concentrated to 5L, stirred overnight and the precipitate was collected by filtration. The solid material was washed with chlorobenzene (2 x 300mL) and dried at 60°C under vacuum overnight. The product was recrystallized from DMF. 68% yield, 99.93% HPLC purity at 292nm. Further purification was achieved by high vacuum sublimation with 85% yield. ESI-MS 534 (M+H).

### Synthesis of 7-(3-(2-(pyridin-2-yl)quinolin-7-yl)phenyl)dibenzo[c,h]acridine (G4):

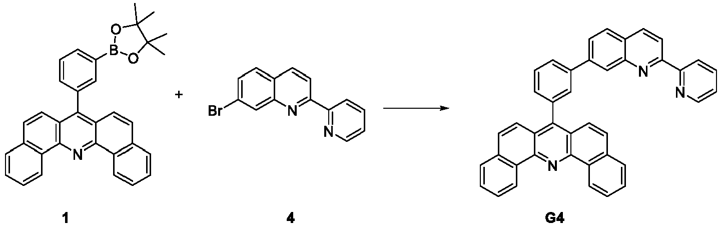

A 3-neck round bottom flask was flushed with nitrogen and charged with 7-(3-(4,4,5,5-tetramethyl-1,3,2-dioxaborolan-2-yl)phenyl)dibenzo[c,h]acridine (22.21g, 46.15mmol, 1eq), 7-bromo-2-(pyridin-2-yl)quinoline (13.16g, 46.15mmol, 1eq), potassium carbonate (15.93g, 115.34mmol, 2.5eq), THF (58 mL), toluene (230 mL) and water (58 mL). The reaction mixture was degassed with N2 for 30 min and the catalyst [1,1'-bis(diphenylphosphino)ferrocene]dichloropalladium(II) (1.01g, 1.38mmol, 0.03eq) was added. The mixture was stirred overnight at 80°C.The reaction mixture was allowed to cool to room temperature and the solvent was removed under vacuum. The raw product was dissolved in DCM and was washed with water (300mL). The organic layer was dried over MgSO4 and filtered through Silica. The solvent was removed and the product was suspended in n-hexane. It was filtered washed and dried again. Then, the product was twice recrystallized from toluene and washed with n-hexane and methanol and dried again. 50% yield, 98.89% HPLC purity at 292nm. Further purification was achieved by high vacuum sublimation with 64% yield. ESI-MS 560 (M+H).

### Bottom emission devices

For bottom emission devices, Examples 1 and 2 and comparative example 1, a 15Ω /cm² glass substrate (available from Corning Co.) with 90 nm ITO was cut to a size of 50 mm x 50 mm x 0.7 mm, ultrasonically washed with isopropyl alcohol for 5 minutes and then with pure water for 5 minutes, and washed again with UV ozone for 30 minutes, to prepare a first electrode. Then, 97 vol.-% Biphenyl-4-yl(9,9-diphenyl-9H-fluoren-2-yl)-[4-(9-phenyl-9H-carbazol-3-yl)phenyl]-amine (CAS 1242056-42-3) with 3 vol.-% 2,2',2"-(cyclopropane-1,2,3-triylidene)tris(2-(p-cyanotetrafluorophenyl)acetonitrile) (A2) was vacuum deposited on the anode, to form a HIL having a thickness of 10 nm according to the inventive Example 1 and 2 and comparative Example 1 (Table 2).

Then, Biphenyl-4-yl(9,9-diphenyl-9H-fluoren-2-yl)-[4-(9-phenyl-9H-carbazol-3-yl) phenyl]-amine was vacuum deposited on the HIL, to form a HTL having a thickness of 128 nm.

Then N,N-bis(4-(dibenzo[b,d]furan-4-yl)phenyl)-[1,1':4',1"-terphenyl]-4-amine (CAS 1198399-61-9) was vacuum deposited on the HTL, to form an electron blocking layer (EBL) having a thickness of 5 nm.

Then 97 vol.-% H09 (Sun Fine Chemicals, South Korea) as EML host and 3 vol.-% BD200 (Sun Fine Chemicals, South Korea) as fluorescent blue dopant were deposited on the EBL, to form a blue-emitting EML with a thickness of 20 nm.

Then the hole blocking layer is formed with a thickness of 5 nm by depositing 2-(3'-(9,9-dimethyl-9H-fluoren-2-yl)-[1,1'-biphenyl]-3-yl)-4,6-diphenyl-1,3,5-triazine on the emission layer.

Then, the electron transporting layer having a thickness of 25 nm is formed on the hole blocking layer by depositing 2-([1,1'-biphenyl]-4-yl)-4-(9,9-diphenyl-9H-fluoren-4-yl)-6-phenyl-1,3,5-triazine. The electron transport layer (ETL) comprises 50 wt.-% matrix compound and 50 wt.-% of LiQ
Then the n-CGL was formed on ETL with a thickness of 15 nm. The n-CGL comprises 99 wt.-% matrix compound of Formula 1 and 1 wt.-% of Li as metal dopant or electron transport layer may comprise 97 wt.-% matrix compound of Formula 1 and 3 wt.-% of Yb, see Table 4. Then the p-CGL was formed on n-CGL with a thickness of 10 nm on n-CGL by depositing Biphenyl-4-yl(9,9-diphenyl-9H-fluoren-2-yl)-[4-(9-phenyl-9H-carbazol-3-yl)phenyl]-amine (CAS 1242056-42-3) with 2,2',2"-(cyclopropane-1,2,3-triylidene)tris(2-(p-cyanotetrafluorophenyl)acetonitrile) (A2) according the inventive example 5-10. (Table 4). In comparative example p-CGL was formed on n-CGL in the same way as in inventive examples accept that HAT-CN was used instead of A2.

Then, Biphenyl-4-yl(9,9-diphenyl-9H-fluoren-2-yl)-[4-(9-phenyl-9H-carbazol-3-yl) phenyl]-amine was vacuum deposited on the p-CGL, to form a second HTL having a thickness of 10 nm.

Al is evaporated at a rate of 0.01 to 1 Å/s at 10⁻⁷ mbar to form a cathode with a thickness of 100 nm.

The OLED stack is protected from ambient conditions by encapsulation of the device with a glass slide. Thereby, a cavity is formed, which includes a getter material for further protection.

To assess the performance of the inventive examples compared to the prior art, the current efficiency is measured at 20°C. The current-voltage characteristic is determined using a Keithley 2635 source measure unit, by sourcing a voltage in V and measuring the current in mA flowing through the device under test. The voltage applied to the device is varied in steps of 0.1V in the range between 0V and 10V. Likewise, the luminance-voltage characteristics and CIE coordinates are determined by measuring the luminance in cd/m² using an Instrument Systems CAS-140CT array spectrometer (calibrated by Deutsche Akkreditierungsstelle (DAkkS)) for each of the voltage values. The cd/A efficiency at 10 mA/cm² is determined by interpolating the luminance-voltage and current-voltage characteristics, respectively.

Lifetime LT of the device is measured at ambient conditions (20°C) and 30 mA/cm², using a Keithley 2400 source meter, and recorded in hours.

The brightness of the device is measured using a calibrated photo diode. The lifetime LT is defined as the time till the brightness of the device is reduced to 97 % of its initial value.

Therefore, it can be seen the material for ETL (Example 1-4) and n-CGL (Example 5-8) can secure low driving voltage and high efficiency of an organic electronic device, when used in an organic electronic device.

In summary, improved operating voltage, which is important for reducing power consumption and increasing battery life, for example of a mobile display device is achieved. At the same time the cd/A efficiency, also referred to as current efficiency is kept at a similar or even improved level. Long life time at high current density is important for the longevity of a device which run at high brightness.

While this invention has been described in connection with what is presently considered to be practical exemplary embodiments, it is to be understood that the invention is not limited to the disclosed embodiments, but, on the contrary, is intended to cover various modifications and equivalent arrangements included within the spirit and scope of the appended claims. Therefore, the aforementioned embodiments should be understood to be exemplary but not limiting the present invention in any way.

In the Table 1 and in Table 2 the properties of inventive compounds of Formula I are listed.

**Table 1**

| Properties of inventive compounds of Formula 1 | | | | | | | |
|---|---|---|---|---|---|---|---|
| Referred to as: | Structure | mp (°C) | Tg (°C) | TRO (°C) | HOMO (eV) | LUMO (eV) | Dipole moment (Debye) |
| G2 | 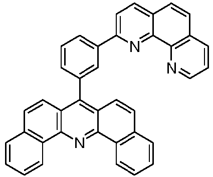 | 319 | 161 | 263 | -5.49 | -1.72 | 4.75 |
| G1 | 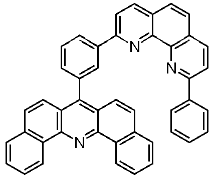 | 303 | 158 | 269 | -5.57 | -1.75 | 3.82 |
| G4 | 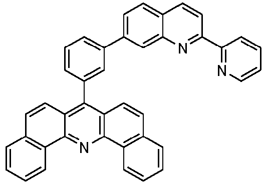 | 247 | 132 | 246 | -5.57 | -1.75 | 4.29 |

**Table 2**

| Performance of an organic electronic device comprising an n-CGL comprising a compound of Formula (I) | | | | | | |
|---|---|---|---|---|---|---|
| Example | Matrix compound | Metal dopant | n-CGL | | Thickness n-CGL (nm) | Operating voltage at 15 mA/cm² (V) |
| | | | Matrix compound (vol.-%) | Metal dopant (vol.-%) | | |
| Example 1 Comparative | 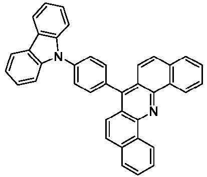 | Li | 99 | 1 | 15 | 11.9 |
| Example 1 G2 | 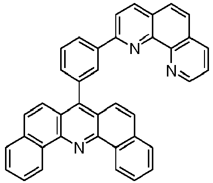 | Li | 99 | 1 | 15 | 4.8 |
| Example 2 G1 | 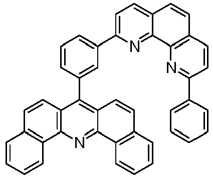 | Li | 99 | 1 | 15 | 4.8 |

In summary, low operating voltage and improved lifetime may be achieved when the organic semiconductor layer comprises a compound of Formula (I).

## Claims

1. A compound represented by the following Formula I:
Ar¹ - L - Ar² (I)
wherein,
Ar¹ is selected from F1 or F2: 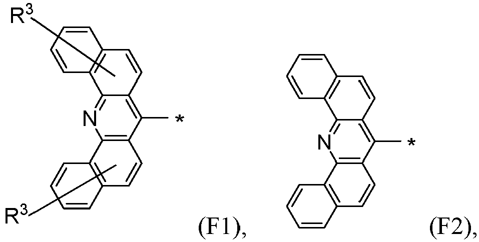 wherein
the asterisk symbol "*" represents the binding position;
R³ may be independently selected from H, C₆ to C₁₈ aryl, C₃ to C₂₀ heteroaryl, C₁ to C₁₆ alkyl, C₁ to C₁₆ alkoxy, C₃ to C₁₆ branched alkyl, C₃ to C₁₆ cyclic alkyl, C₃ to C₁₆ branched alkoxy, C₃ to C₁₆ cyclic alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy, -PX¹(R²)₂, D, F or CN;
wherein R² is independently selected from C₆ to C₁₂ aryl, C₃ to C₁₂ heteroaryl, C₁ to C₁₆ alkyl, C₁ to C₁₆ alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy;
X¹ is selected from S or O;
L is selected from substituted or unsubstituted phenylene, a substituted or unsubstituted biphenylene, a substituted or unsubstituted terphenylene, a substituted or unsubstituted anthracenylene, a substituted or unsubstituted naphthylene, a substituted or unsubstituted dibenzofuranylene, a substituted or unsubstituted dibenzothiophenylene, a substituted or unsubstituted carbazolylene, a substituted or unsubstituted pyridinylene, a substituted or unsubstituted phenylpyridinylene, a substituted or unsubstituted quinolinylene; and
Ar² has the Formula IIa, IIc, IId or IIe, wherein the asterisk symbol "*" represents the binding position: 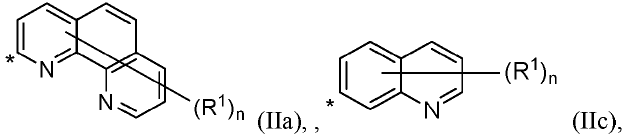 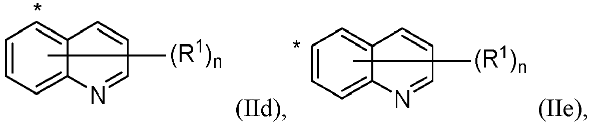 wherein
R¹ and substituents of Ar¹ and L are independently selected from H, C₆ to C₁₈ aryl, C₃ to C₂₀ heteroaryl, C₁ to C₁₆ alkyl, C₁ to C₁₆ alkoxy, C₃ to C₁₆ branched alkyl, C₃ to C₁₆ cyclic alkyl, C₃ to C₁₆ branched alkoxy, C₃ to C₁₆ cyclic alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy, -PX¹(R²)₂, D, F or CN; wherein
R² is independently selected from C₆ to C₁₂ aryl, C₃ to C₁₂ heteroaryl, C₁ to C₁₆ alkyl, C₁ to C₁₆ alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy;
X¹ is selected from S or O; and
n is 0, 1, 2 or 3; and
wherein Formula I comprises at least 9 to 25 aromatic rings.

2. The compound of Formula 1 according to claim 1, wherein the compound of Formula 1 is represented by the following Formula Ib:

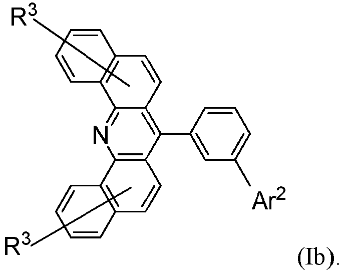

3. The compound of Formula 1 according to claim 1 or 2, wherein Ar² is selected from E1 to E4: 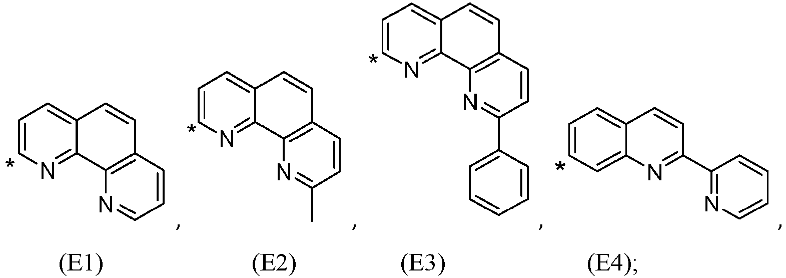 wherein, the asterisk symbol "*" represents the binding position.

4. The compound of Formula 1 according to any of the preceding claims 1 to 3, wherein L is linked with Ar¹ and Ar² and selected from the group comprising an unsubstituted phenylene, an unsubstituted biphenylene, an unsubstituted terphenylene, an unsubstituted anthracenylene, an unsubstituted naphthylene, an unsubstituted dibenzofuranylene, an unsubstituted dibenzothiophenylene, an unsubstituted carbazolylene, an unsubstituted pyridinylene, an unsubstituted phenylpyridinylene, an unsubstituted quinolinylene.

5. The compound of Formula 1 according to any of the preceding claims 1 to 4, wherein L is selected from B1 to B21: 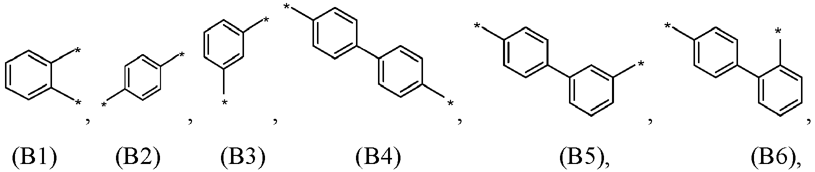 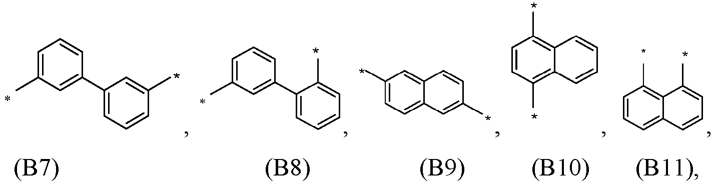 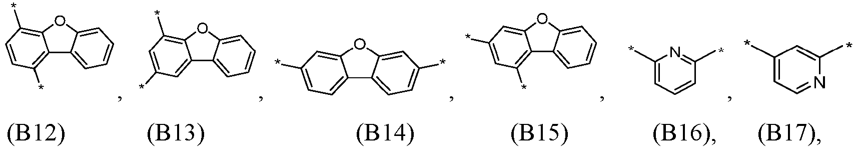 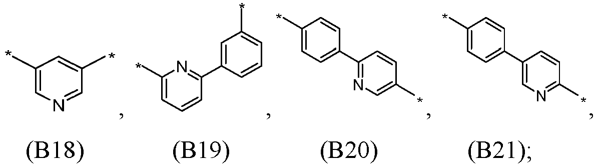 wherein the asterisk symbol "*" represents the binding position.

6. The compound of Formula 1 according to any of the preceding claims 1 to 5, wherein R¹ is selected from D1 to D8: 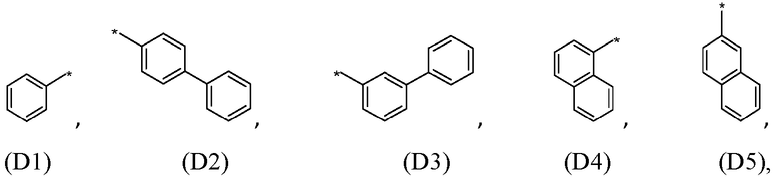 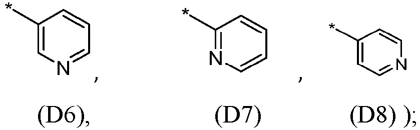 wherein the asterisk symbol "*" represents the binding position.

7. The compound of Formula 1 according to any of the preceding claims 1 to 6, wherein the compound of formula (I) are selected from G1 to G20:

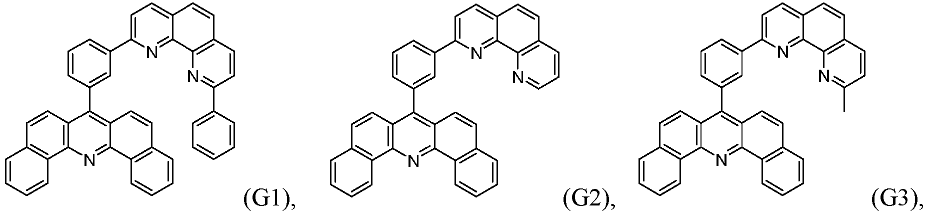

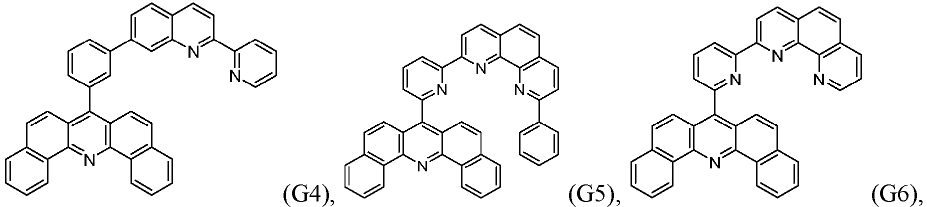

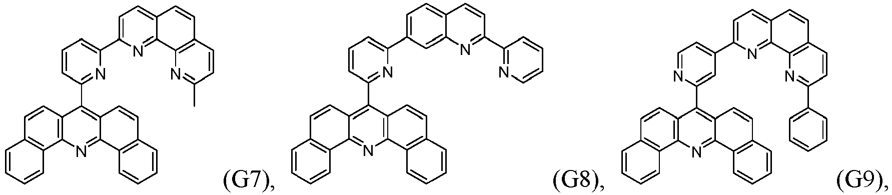

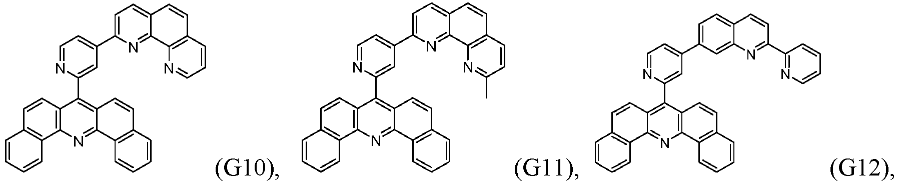

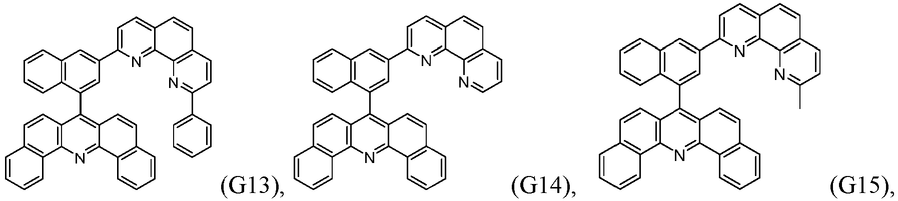

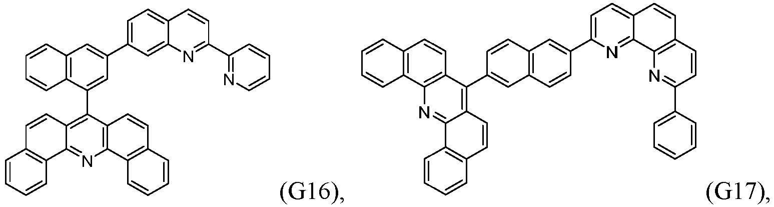

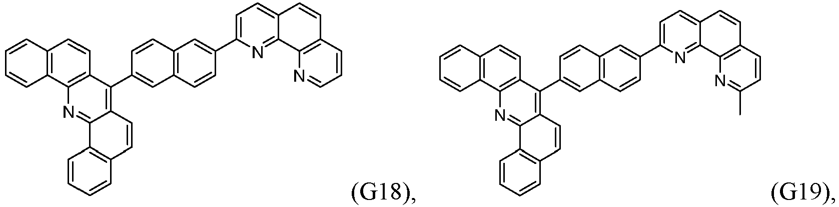

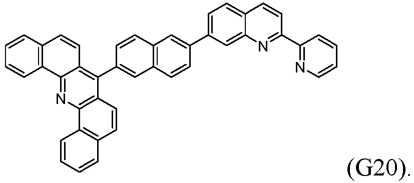

8. An organic semiconductor layer that comprises at least one compound represented by Formula I according to any of the preceding claims 1 to 7.

9. The organic semiconductor layer according to claim 8, wherein the organic semiconductor layer further comprises a dopant, wherein the dopant is selected from the group comprising a metal, metal salt or organic metal complex.

10. An organic electronic device comprising an anode layer, a cathode layer and at least one organic semiconductor layer according to claim 8 or 9, wherein the at least one organic semiconductor layer comprises a compound of formula 1 according to any of the preceding claims 1 to 7.

11. The organic electronic device according to claim 10, wherein the organic semiconductor layer is an n-type charge generation layer.

12. The organic electronic device according to claim 10 or 11 further comprising at least one emission layer, wherein the organic semiconductor layer is arranged between the at least one emission layer and the cathode layer.

13. The organic electronic device according to any of the preceding claims 10 to 12, wherein the electronic device is a light emitting device, thin film transistor, a battery, a display device or a photovoltaic cell, and preferably a light emitting device.

## Patentansprüche

1. Verbindung, die durch die folgende Formel I wiedergegeben wird:
Ar¹ - L - Ar² (I)
wobei
Ar¹ aus F1 oder F2 ausgewählt ist: 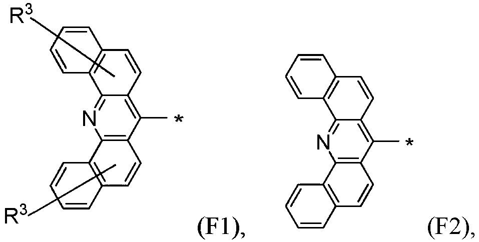 wobei
das Sternchensymbol "*" die Anbindungsstelle repräsentiert;
R³ unabhängig aus H, C₆- bis C₁₈-Aryl, C₃- bis C₂₀-Heteroaryl, C₁- bis C₁₆-Alkyl, C₁- bis C₁₆-Alkoxy, verzweigtem C₃- bis C₁₆-Alkyl, cyclischem C₃- bis C₁₆-Alkyl, verzweigtem C₃- bis C₁₆-Alkoxy, cyclischem C₃- bis C₁₆-Alkoxy, teil- oder perfluoriertem C₁- bis C₁₆-Alkyl, teil- oder perfluoriertem C₁- bis C₁₆-Alkoxy, teil- oder perdeuteriertem C₁- bis C₁₆-Alkyl, teil- oder perdeuteriertem C₁- bis C₁₆-Alkoxy, -PX¹(R²)₂, D, F oder CN ausgewählt sein kann;
wobei R² unabhängig aus C₆- bis C₁₂-Aryl, C₃- bis C₁₂-Heteroaryl, C₁- bis C₁₆-Alkyl, C₁- bis C₁₆-Alkoxy, teil-oder perfluoriertem C₁- bis C₁₆-Alkyl, teil- oder perfluoriertem C₁- bis C₁₆-Alkoxy, teil- oder perdeuteriertem C₁- bis C₁₆-Alkyl, teil- oder perdeuteriertem C₁- bis C₁₆-Alkoxy ausgewählt ist;
X¹ aus S oder O ausgewählt ist;
L aus substituiertem oder unsubstituiertem Phenylen, einem substituierten oder unsubstituierten Biphenylen, einem substituierten oder unsubstituierten Terphenylen, einem substituierten oder unsubstituierten Anthracenylen, einem substituierten oder unsubstituierten Naphthylen, einem substituierten oder unsubstituierten Dibenzofuranylen, einem substituierten oder unsubstituierten Dibenzothiophenylen, einem substituierten oder unsubstituierten Carbazolylen, einem substituierten oder unsubstituierten Pyridinylen, einem substituierten oder unsubstituierten Phenylpyridinylen, einem substituierten oder unsubstituierten Chinolinylen ausgewählt ist; und
Ar² die Formel IIa, IIc, IId oder IIe aufweist, wobei das Sternchensymbol "*" die Anbindungsstelle repräsentiert: 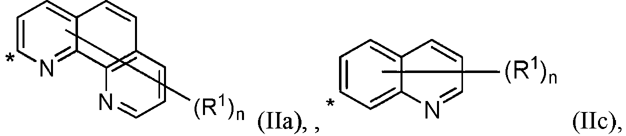 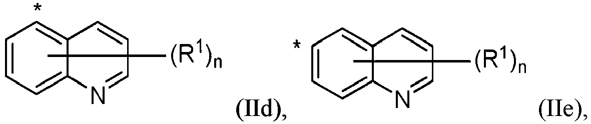 wobei
R¹ und Substituenten von Ar¹ und L unabhängig aus H, C₆- bis C₁₈-Aryl, C₃- bis C₂₀-Heteroaryl, C₁- bis C₁₆-Alkyl, C₁- bis C₁₆-Alkoxy, verzweigtem C₃- bis C₁₆-Alkyl, cyclischem C₃- bis C₁₆-Alkyl, verzweigtem C₃- bis C₁₆-Alkoxy, cyclischem C₃- bis C₁₆-Alkoxy, teil- oder perfluoriertem C₁- bis C₁₆-Alkyl, teil- oder perfluoriertem C₁- bis C₁₆-Alkoxy, teil- oder perdeuteriertem C₁- bis C₁₆-Alkyl, teil- oder perdeuteriertem C₁- bis C₁₆-Alkoxy, -PX¹(R²)₂, D, F oder CN ausgewählt sind; wobei
R² unabhängig aus C₆- bis C₁₂-Aryl, C₃- bis C₁₂-Heteroaryl, C₁- bis C₁₆-Alkyl, C₁- bis C₁₆-Alkoxy, teil- oder perfluoriertem C₁- bis C₁₆-Alkyl, teil- oder perfluoriertem C₁- bis C₁₆-Alkoxy, teil- oder perdeuteriertem C₁- bis C₁₆-Alkyl, teil- oder perdeuteriertem C₁- bis C₁₆-Alkoxy ausgewählt ist;
X¹ aus S oder O ausgewählt ist; und
n für 0, 1, 2 oder 3 steht; und
wobei Formel I mindestens 9 bis 25 aromatische Ringe umfasst.

2. Verbindung der Formel 1 nach Anspruch 1, wobei die Verbindung der Formel 1 durch die folgende Formel Ib wiedergegeben wird:

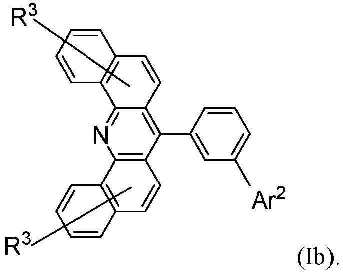

3. Verbindung der Formel 1 nach Anspruch 1 oder 2, wobei Ar² aus E1 bis E4 ausgewählt ist: 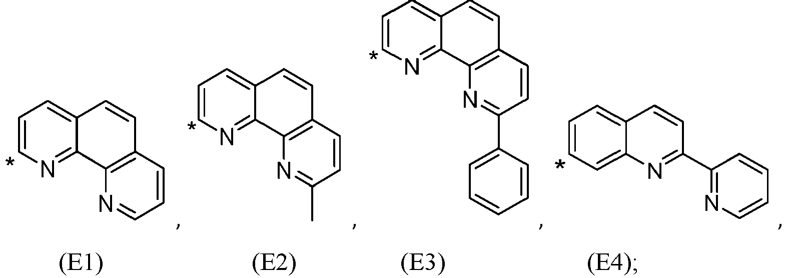 wobei das Sternchensymbol "*" die Anbindungsstelle repräsentiert.

4. Verbindung der Formel 1 nach einem der vorhergehenden Ansprüche 1 bis 3, wobei L mit Ar¹ und Ar² verknüpft ist und aus der Gruppe umfassend ein unsubstituiertes Phenylen, ein unsubstituiertes Biphenylen, ein unsubstituiertes Terphenylen, ein unsubstituiertes Anthracenylen, ein unsubstituiertes Naphthylen, ein unsubstituiertes Dibenzofuranylen, ein unsubstituiertes Dibenzothiophenylen, ein unsubstituiertes Carbazolylen, ein unsubstituiertes Pyridinylen, ein unsubstituiertes Phenylpyridinylen, ein unsubstituiertes Chinolinylen ausgewählt ist.

5. Verbindung der Formel 1 nach einem der vorhergehenden Ansprüche 1 bis 4, wobei L aus B1 bis B21 ausgewählt ist: 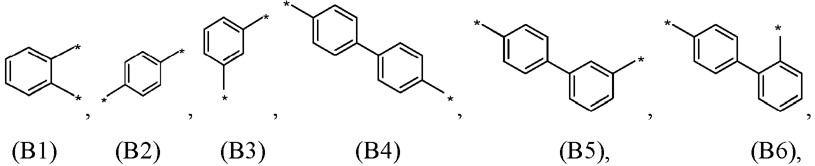 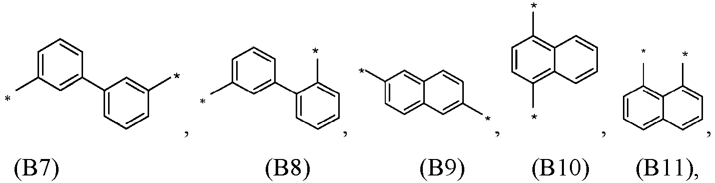 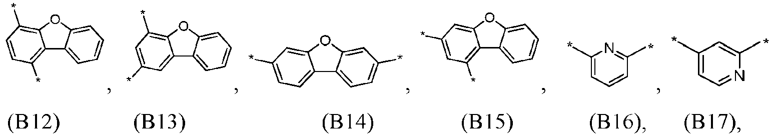 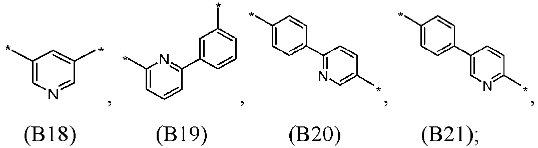 wobei das Sternchensymbol "*" die Anbindungsstelle repräsentiert.

6. Verbindung der Formel 1 nach einem der vorhergehenden Ansprüche 1 bis 5, wobei R¹ aus D1 bis D8 ausgewählt ist: 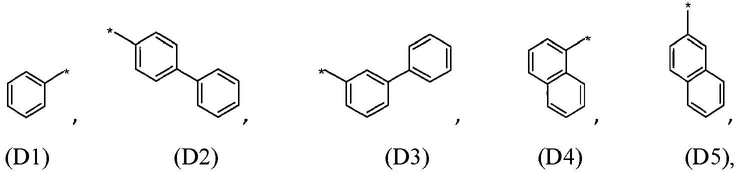 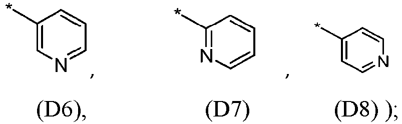 wobei das Sternchensymbol "*" die Anbindungsstelle repräsentiert.

7. Verbindung der Formel 1 nach einem der vorhergehenden Ansprüche 1 bis 6, wobei die Verbindung der Formel (I) aus G1 bis G20 ausgewählt ist:

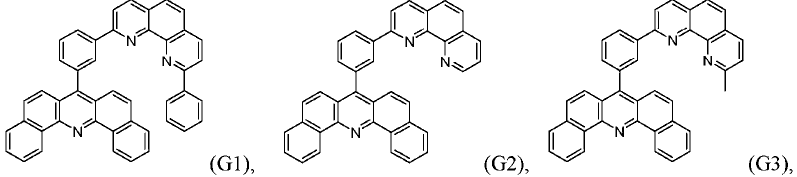

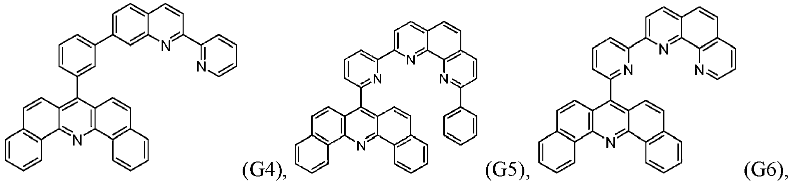

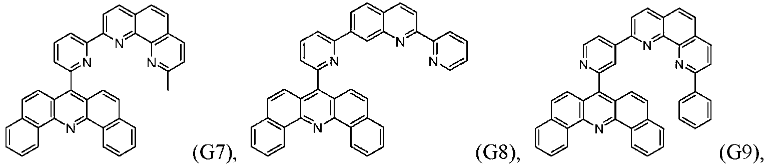

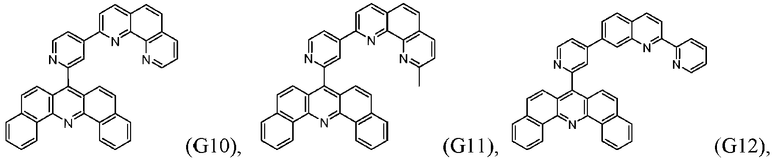

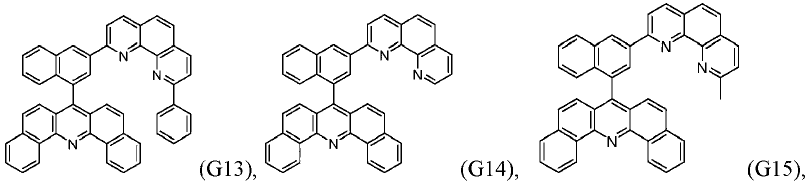

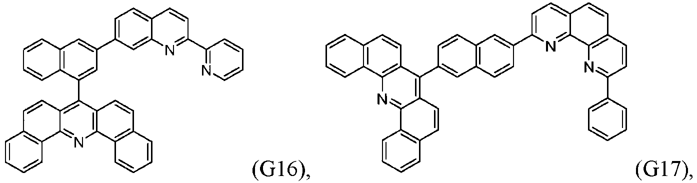

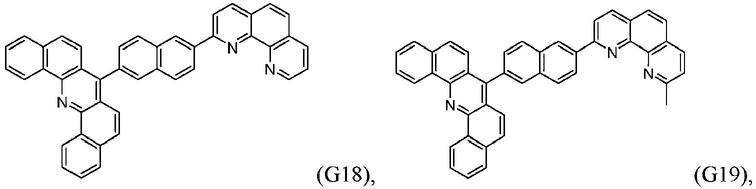

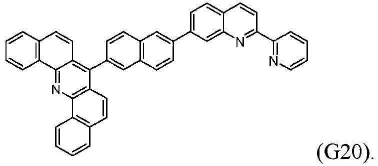

8. Organische Halbleiterschicht, die mindestens eine durch Formel I wiedergegebene Verbindung nach einem der vorhergehenden Ansprüche 1 bis 7 umfasst.

9. Organische Halbleiterschicht nach Anspruch 8, wobei die organische Halbleiterschicht ferner einen Dotierstoff umfasst, wobei der Dotierstoff aus der Gruppe umfassend ein Metall, ein Metallsalz oder einen organischen Metallkomplex ausgewählt ist.

10. Organische elektronische Vorrichtung, umfassend eine Anodenschicht, eine Kathodenschicht und mindestens eine organische Halbleiterschicht nach Anspruch 8 oder 9, wobei die mindestens eine organische Halbleiterschicht eine Verbindung der Formel 1 nach einem der vorhergehenden Ansprüche 1 bis 7 umfasst.

11. Organische elektronische Vorrichtung nach Anspruch 10, wobei es sich bei der organischen Halbleiterschicht um eine Ladungserzeugungsschicht vom n-Typ handelt.

12. Organische elektronische Vorrichtung nach Anspruch 10 oder 11, die ferner mindestens eine Emissionsschicht umfasst, wobei die organische Halbleiterschicht zwischen der mindestens einen Emissionsschicht und der Kathodenschicht angeordnet ist.

13. Organische elektronische Vorrichtung nach einem der vorhergehenden Ansprüche 10 bis 12, wobei es sich bei der elektronischen Vorrichtung um eine lichtemittierende Vorrichtung, einen Dünnschichttransistor, eine Batterie, eine Anzeigevorrichtung oder eine Photovoltaikzelle und vorzugsweise eine lichtemittierende Vorrichtung handelt.

## Revendications

1. Composé représenté par la formule I suivante :
Ar¹ - L - Ar² (I)
dans laquelle,
Ar¹ est choisi parmi F1 ou F2 : 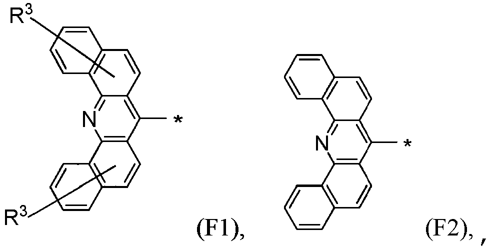 dans lesquelles
le symbole astérisque « * » représente la position de liaison ;
R³ peut être indépendamment choisi parmi H, C₆ à C₁₈ aryle, C₃ à C₂₀ hétéroaryle, C₁ à C₁₆ alkyle, C₁ à C₁₆ alcoxy, C₃ à C₁₆ alkyle ramifié, C₃ à C₁₆ alkyle cyclique, C₃ à C₁₆ alcoxy ramifié, C₃ à C₁₆ alcoxy cyclique, C₁ à C₁₆ alkyle partiellement ou perfluoré, C₁ à C₁₆ alcoxy partiellement ou perfluoré, C₁ à C₁₆ alkyle partiellement ou perdeutéré, C₁ à C₁₆ alcoxy partiellement ou perdeutéré, -PX¹(R²)₂, D, F ou CN ;
dans lesquelles R² est indépendamment choisi parmi C₆ à C₁₂ aryle, C₃ à C₁₂ hétéroaryle, C₁ à C₁₆ alkyle, C₁ à C₁₆ alcoxy, C₁ à C₁₆ alkyle partiellement ou perfluoré, C₁ à C₁₆ alcoxy partiellement ou perfluoré, C₁ à C₁₆ alkyle partiellement ou perdeutéré, C₁ à C₁₆ alcoxy partiellement ou perdeutéré ;
X¹ est choisi parmi S ou O ;
L est choisi parmi phénylène substitué ou non substitué, un biphénylène substitué ou non substitué, un terphénylène substitué ou non substitué, un anthracénylène substitué ou non substitué, un naphtylène substitué ou non substitué, un dibenzofuranylène substitué ou non substitué, un dibenzothiophénylène substitué ou non substitué, un carbazolylène substitué ou non substitué, un pyridinylène substitué ou non substitué, un phénylpyridinylène substitué ou non substitué, un quinoléinylène substitué ou non substitué ; et
Ar² a la formule IIa, IIc, IId ou IIe, dans laquelle le symbole astérisque « * » représente la position de liaison : 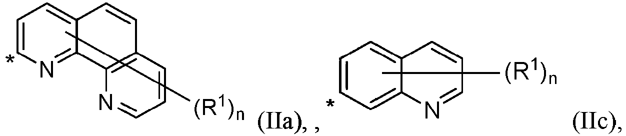 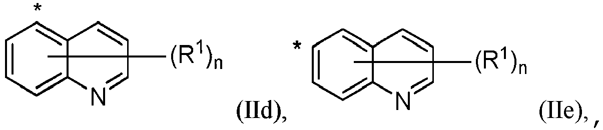 dans lesquelles
R¹ et des substituants de Ar¹ et L sont indépendamment choisis parmi H, C₆ à C₁₈ aryle, C₃ à C₂₀ hétéroaryle, C₁ à C₁₆ alkyle, C₁ à C₁₆ alcoxy, C₃ à C₁₆ alkyle ramifié, C₃ à C₁₆ alkyle cyclique, C₃ à C₁₆ alcoxy ramifié, C₃ à C₁₆ alcoxy cyclique, C₁ à C₁₆ alkyle partiellement ou perfluoré, C₁ à C₁₆ alcoxy partiellement ou perfluoré, C₁ à C₁₆ alkyle partiellement ou perdeutéré, C₁ à C₁₆ alcoxy partiellement ou perdeutéré, -PX¹(R²)₂, D, F ou CN ; dans laquelle
R² est indépendamment choisi parmi C₆ à C₁₂ aryle, C₃ à C₁₂ hétéroaryle, C₁ à C₁₆ alkyle, C₁ à C₁₆ alcoxy, C₁ à C₁₆ alkyle partiellement ou perfluoré, C₁ à C₁₆ alcoxy partiellement ou perfluoré, C₁ à C₁₆ alkyle partiellement ou perdeutéré, C₁ à C₁₆ alcoxy partiellement ou perdeutéré ;
X¹ est choisi parmi S ou O ; et
n est 0, 1, 2 ou 3 ; et
dans laquelle la formule I comprend au moins 9 à 25 cycles aromatiques.

2. Composé de formule 1 selon la revendication 1, dans lequel le composé de formule 1 est représenté par la formule suivante Ib:

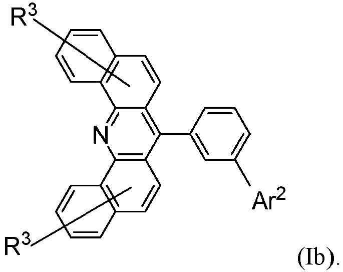

3. Composé de formule 1 selon la revendication 1 ou 2, dans lequel Ar² est choisi parmi E1 à E4: 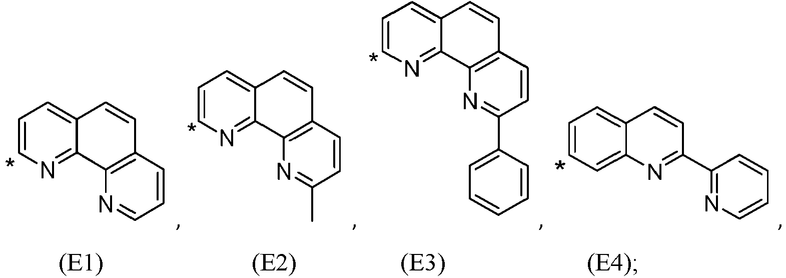 dans lesquels, le symbole astérisque « * » représente la position de liaison.

4. Composé de formule 1 selon l'une quelconque des revendications 1 à 3, dans lequel L est lié avec Ar¹ et Ar² et choisi dans le groupe comprenant un phénylène non substitué, un biphénylène non substitué, un terphénylène non substitué, un anthracénylène non substitué, un naphtylène non substitué, un dibenzofuranylène non substitué, un dibenzothiophénylène non substitué, un carbazolylène non substitué, un pyridinylène non substitué, un phénylpyridinylène non substitué, un quinoléinylène non substitué.

5. Composé de formule 1 selon l'une quelconque des revendications 1 à 4, dans lequel L est choisi parmi B1 à B21: 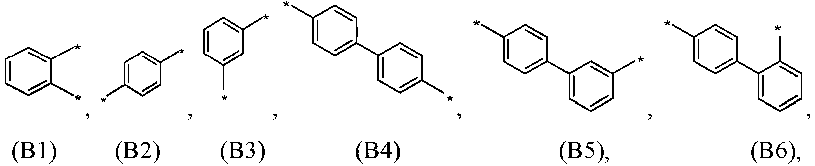 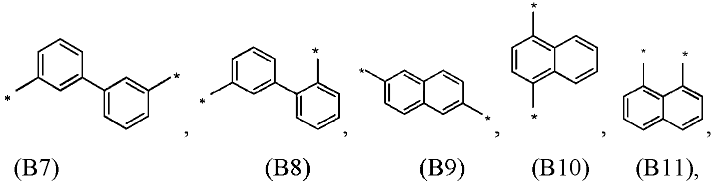 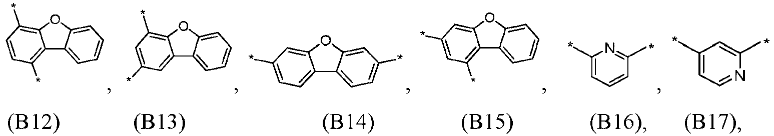 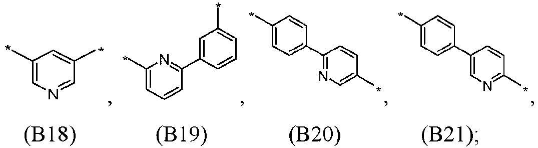 dans lequel le symbole astérisque « * » représente la position de liaison.

6. Composé de formule 1 selon l'une quelconque des revendications 1 à 5, dans lequel R¹ est choisi parmi D1 à D8: 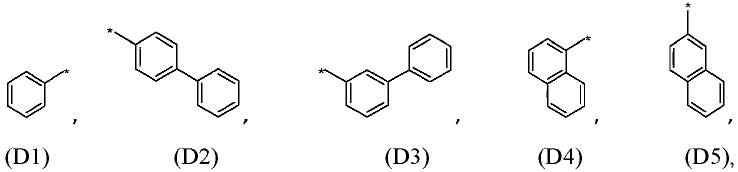 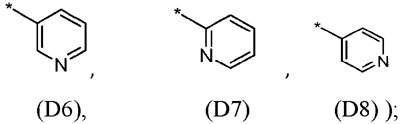 dans lequel le symbole astérisque « * » représente la position de liaison.

7. Composé de formule 1 selon l'une quelconque des revendications 1 à 6, dans lequel le composé de formule (I) est choisi parmi G1 à G20:

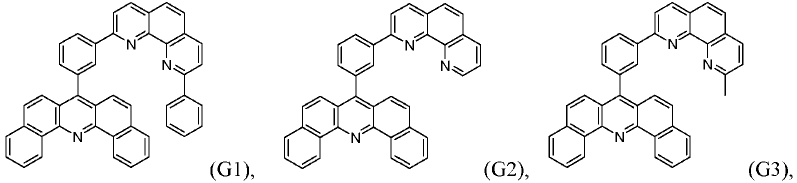

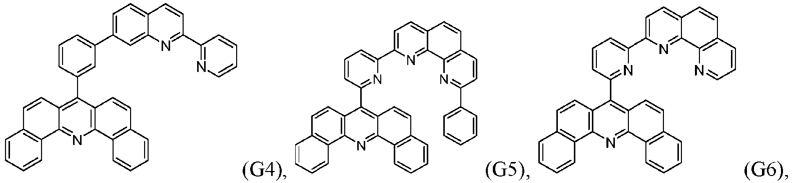

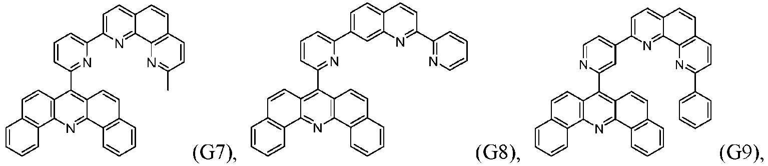

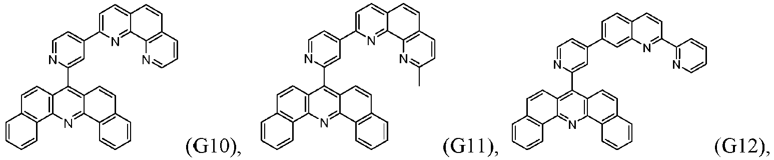

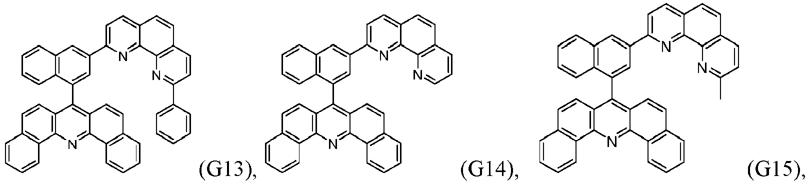

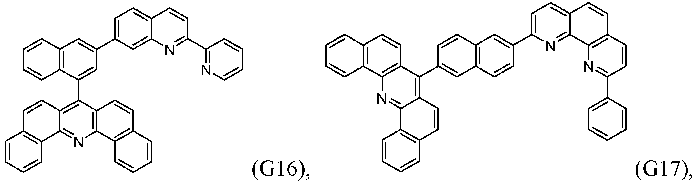

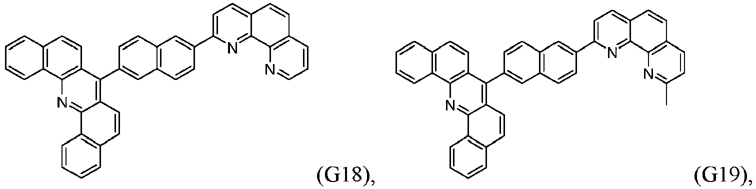

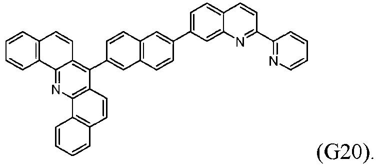

8. Couche de semi-conducteur organique qui comprend au moins un composé représenté par la formule I selon l'une quelconque des revendications précédentes 1 à 7.

9. Couche de semi-conducteur organique selon la revendication 8, dans laquelle la couche de semi-conducteur organique comprend en outre un dopant, dans laquelle le dopant est choisi dans le groupe comprenant un métal, un sel métallique ou un complexe métallique organique.

10. Dispositif électronique organique comprenant une couche d'anode, une couche de cathode et au moins une couche de semi-conducteur organique selon la revendication 8 ou 9, dans lequel l'au moins une couche de semi-conducteur organique comprend un composé de formule 1 selon l'une quelconque des revendications précédentes 1 à 7.

11. Dispositif électronique organique selon la revendication 10, dans lequel la couche de semi-conducteur organique est une couche de génération de charge de type n.

12. Dispositif électronique organique selon la revendication 10 ou 11, comprenant en outre au moins une couche d'émission, dans lequel la couche de semi-conducteur organique est agencée entre l'au moins une couche d'émission et la couche de cathode.

13. Dispositif électronique organique selon l'une quelconque des revendications précédentes 10 à 12, dans lequel le dispositif électronique est un dispositif émetteur de lumière, un transistor en couche mince, une batterie, un dispositif d'affichage ou une cellule photovoltaïque, et préférablement un dispositif émetteur de lumière.
